# EUROPEAN PATENT SPECIFICATION

(11) **EP 1 664 121 B1**
(45) Date of publication and mention of the grant of the patent: **08.07.2009**
(21) Application number: 04764984.3
(22) Date of filing: 09.09.2004
(51) Int. Cl.: C07K 16/28, C12N 5/10, C12N 15/13, C12N 5/12

(54) **ANTIBODIES AGAINST INTERLEUKIN-1 RECEPTOR AND USES THEREOF**
ANTI-INTERLEUKIN-1 REZEPTOR ANTIKÖRPER UND DEREN VERWENDUNG
ANTICORPS CONTRE LE RECEPTEUR INTERLEUKINE-1 ET LEURS UTILISATIONS

(30) Priority: 10.09.2003 US 501681 P; 23.12.2003 EP 03029659
(43) Date of publication of application: 07.06.2006
(73) Proprietor: F.HOFFMANN-LA ROCHE AG, 4070 Basel (CH)
(72) Inventor: BARTKE, Ilse, 82347 Bernried (DE); CARR, Francis, Aberdeenshire AB23 8XU (GB); CHIZZONITE, Anthony, South Kent, CT 06785 (US); EUGUI, Elsie, Belmont, CA 94002 (US); FERTIG, Georg, 82377 Penzberg (DE); HAMILTON, Anita, Aberdeen AB15 4EQ (GB); LANZENDOERFER, Martin, 82327 Tutzing (DE); RUEGER, Petra, 82377 Penzberg (DE); SCHUMACHER, Ralf, 82377 Penzberg (DE); TRUITT, Theresa, Bloomfield, NJ 07003 (US)
(74) Representative: Schreiner, Siegfried
(86) International application number: PCT/EP2004/010047
(87) International publication number: WO 2005/023872

(56) References cited:
- WO-A-02/11767
- CHOE JUNG-YOON ET AL: "Interleukin 1 receptor dependence of serum transferred arthritis can be circumvented by toll-like receptor 4 signaling." JOURNAL OF EXPERIMENTAL MEDICINE, vol. 197, no. 4, 17 February 2003 (2003-02-17), pages 537-542, XP002309814 ISSN: 0022-1007
- BD PHARMINGEN: "Purified rat anti-mouse CD121a (IL-1 receptor, type I, p80) monoclonal antibody (no azide/low endotoxin)" TECHNICAL DATA SHEET, [Online] June 2003 (2003-06), XP002309815 Retrieved from the Internet: <URL:www.abcam.com/?datasheet=5405> [retrieved on 2004-12-09]
- ABCAM: "Telomerase antibody [2D8] (ab5405) datasheet" INTERNET ARTICLE, [Online] XP002309816 Retrieved from the Internet: <URL:www.abcam.com/?datasheet=5405> [retrieved on 2004-12-09]
- ABCAM: "IL1 beta antibody [2D8] (ab13775) datasheet" INTERNET ARTICLE, [Online] XP002309817 Retrieved from the Internet: <URL:www.abcam.com/index.html?datasheet=13 775> [retrieved on 2004-12-09]

## Description

The present invention relates to antibodies against interleukin-1 receptor(IL-1R), methods for their production, pharmaceutical compositions containing said antibodies, and uses thereof.

The signal transduction pathways activated by the proinflammatory cytokine interleukin-1 (IL-lalpha and IL-1beta) have been the focus of much attention because of the important role that IL-1 plays in inflammatory diseases and is involved in the inflammation and the joint destruction associated with rheumatoid arthritis.

A number of proteins have been described that participate in the post-receptor activation of the transcription factor nuclear factor kappaB (NF-kappaB), and stress-activated protein kinases such as p38 mitogen-activated protein kinase (MAPK). Interleukin-1 receptor(IL-1R, Swiss Prot. P14778, CD 121a) is a member of this signaling system which is a critical determinant of the innate immune and inflammatory response. The treatment approach to rheumatoid arthritis has undergone a major evolutionary change in recent years in part as a consequence of growing appreciation of the severity of this condition and in part due to very considerable progress in understanding the important role of cytokines in the immunopathogenesis of this disease. The major focus is based on the rationale for targeting TNFα and IL-1. Recently published studies confirm that the use of a several biological agents targeting TNFα give rise to sustained improvements in symptoms and signs of rheumatoid disease and, furthermore; that TNFα blockade protects joints from structural damage. Anakinra is an interleukin-1 receptor antagonist (IL-1ra), which blocks actions mediated by IL-1.

US Patent No. 6,511,665 claims a monoclonal antibody which specifically binds to a human IL-1 receptor and blocks binding of IL-1 to IL-1 receptor.

The object of the invention is to provide novel antibodies against IL-1R which are valuable means for a therapy of inflammatory diseases like rheumatoid arthritis.

### Summary of the Invention

It was surprisingly found that there exist antibodies against IL-1R which show an IC-50 value of 35 pM or lower for the inhibition of IL-1 mediated secretion of IL-8 (in human embryonic lung fibroblasts such as MRC-5 cells).

Antibodies according to the invention have an epitope specificity for IL-1R on native and denatured IL-1R and inhibit the binding of IL-1 to IL-1R and the subsequent signal transduction (activation of nuclear factor kappa B (NF-kappaB). The antibodies bind to the soluble domain of IL-1R in its glycosylated form and show an affinity of 300 pM, preferably 200 pM or less (K_{D}). The antibodies show a significantly reduced affinity for the binding to deglycosylated IL-1R.

Antibodies according to the invention inhibit the binding of IL-1 to IL-1R in vitro and in vivo and therefore inhibit forming of a ternary complex consisting of IL-1, IL-1 receptor and IL-1Racp (Interleukin-1 receptor accessory protein; Q9NPH3).

The invention comprises an antibody binding to human IL-1R and inhibiting the binding of human IL-1 to IL-1R, characterized in that said antibody is preferably obtainable from hybridoma cell line MAK<h-IL-1RI>2D8 (DSM ACC 2601) or is a chimeric, humanized or T cell epitope depleted antibody variant or a fragment of said antibody, showing an IC-50 value of 35 pM or lower for the inhibition of IL-1 mediated secretion of II-8 in human fibroblast cells MRC5 (ATCC CCL 171).

Antibodies according to the invention preferably do not show effector function (ADCC and CDC) and are therefore of IgG4 isotype. Especially preferred is mutation of serine 228 to proline (Angal, S., et al., Mol. Immunol. 30 (1993) 105-108). Alternatively said antibodies are of IgG1 isotype and preferably modified in the hinge region at about aa 220-240 between CH1 and CH2 and/or the second inter-domain region of about aa 330 between CH2 and CH3 (numbering according to Kabat, see e.g. Johnson, G., and Wu, T.T., Nucleic Acids Res. 28 (2000) 214-218) to avoid effector function. Switching of IgG class can be easily performed by exchange of the constant heavy and light chains of the antibody by heavy and light chains from an antibody of the desired class, like IgG₁ or IgG₄. Such methods are well known in the state of the art.

Antibodies according to the invention show benefits for patients in need of anti inflammatory therapy. The antibodies according to the invention have new and inventive properties causing a benefit for a patient suffering from such a disease, especially sufffering from rheumatoid arthritis. The antibodies according to the invention are characterized by the above mentioned properties.

It is further preferred that the antibody is of rat origin and comprises the antibody sequence frame of a rat antibody according to Kabat. Preferably in the Kabat sequences amino acid 10 (serine) is deleted from the VL chain (DEL10) and/or amino acid 26 (glycine) of the VH chain is changed to glutamic acid (G26E). Preferaly the antibody is T cell epitope depleted using methods described in WO 98/08097.

It is further preferred that IL-1R antagonist (Arend W.P., J. Clin. Invest. 88 (1991) 1445-1451) do not inhibit binding of IL-1R (10nM) to an immobilized antibody according to the invention in a concentration of up to 100 µM (IL-1R antagonist) i.e. binding of an antibody according to the invention to IL-1R is not inhibited by II-1R antagonist.

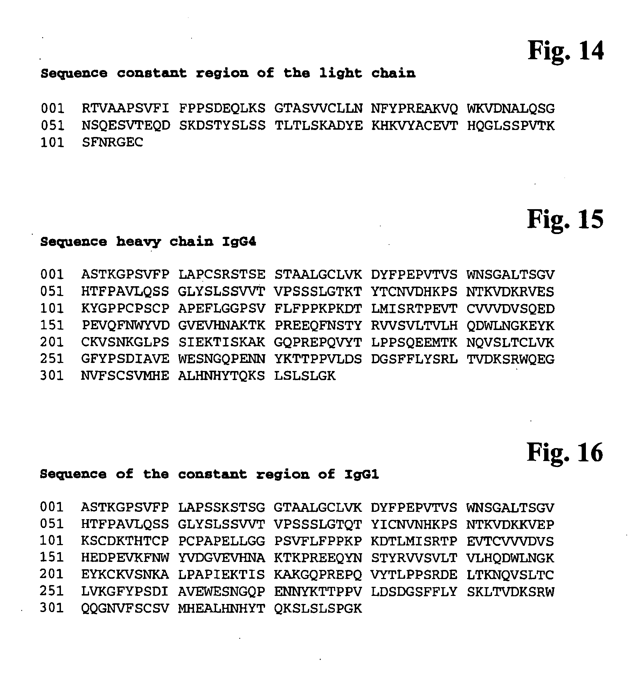

The constant region is preferably a human IgG1 or human IgG4 constant region according to Kabat, E. (see below). Preferred constant regions are shown in Fig 14, 15 and 16.

The invention also comprises antibody encoding nucleic acids. The encoded polypeptides are capable of assembling together with the respective other antibody chain defined below:
an antibody heavy chain comprising as CDRs CDR1 (aa 45-54), CDR2 (aa 69-84) and CDR3 (aa 117-123) of SEQ ID NO:1, an antibody light chain comprising as CDRs CDR1 (aa 43-57), CDR2 (aa 73-79) and CDR3 (aa 112-120) of SEQ ID NO:2. The CDR numbering and definition is according to Kabat, E. (see e.g. Johnson, G., and Wu, T.T., Nucleic Acids Res. 28 (2000) 214-218) including the signal sequences.

Preferably, the nucleic acid encodes a polypeptide which is a heavy chain consisting of a variable region (VH) of SEQ ID NO:1 and a light chain consisting of a variable region (VL) of SEQ ID NO:2.

The antibody is preferably a monoclonal antibody and, in addition, a chimeric antibody (human constant chain), a humanized antibody and especially preferably a T cell epitope depleted antibody.

The antibody binds to IL-1R human in competition to the antibodies characterized by the variable chains of SEQ ID NOS:1-2.

The antibody is further characterized by an affinity of 300 pM or less, preferably 200 pM (K_{D}) or less and more preferably of about 70-200 pM.

The invention therefore comprises also a polypeptide and an encoding nucleic acid selected from the above-mentioned group consisting of CDR1, CDR2, CDR3 of heavy chain and CDR1, CDR2, CDR3 of light chain of an IL-1R antibody according to the invention.

The invention further provides hybridoma cell lines which produce such antagonistic monoclonal antibodies according to the invention.

The preferred hybridoma cell line according to the invention, hybridoma cell line MAK<h-IL-1RI>2D8 was deposited, under the Budapest Treaty on the international recognition of the deposit of microorganisms for the purpose of patent procedure, with Deutsche Sammlung von Mikroorganismen und Zellkulturen GmbH (DSMZ), Germany, on July 10, 2003 under Accession No. DSM ACC 2601.

An antibody obtainable from said cell line is a preferred embodiment of the invention. Preferred are also all antibodies which can be combined from the variable and constant regions shown in Figures 7 to 10 and 14 to 16 and showing an IC-50 value of 35 pM or lower for the inhibition of IL-1 mediated secretion of 11-8 in human fibroblast cells MRC5 (ATCC CCL 171). These sequences are examples of sequences which were obtained by modifying the sequence of antibody 2D8 in order to get improved antibodies retaining the superior properties of antibody 2D8 which are IC50 and/or epitope characteristics. In such antibodies, light chains and heavy chains from Figs. 7 and 8 (T cell epitope depleted) or from Figs. 9 and 10 (humanized) are combined with constant region from Fig. 14 and Fig. 15 or 16. Especially preferred are antibodies DEI5/7, DEI4/7, DEI2/4, DEI5/4, DEI4/5, DEI5/5, HUM2/2, HUM2/3, DEI1/8, DEI2/8, DEI2/9, DEI4/9, DEI5/8 and DE15/9.

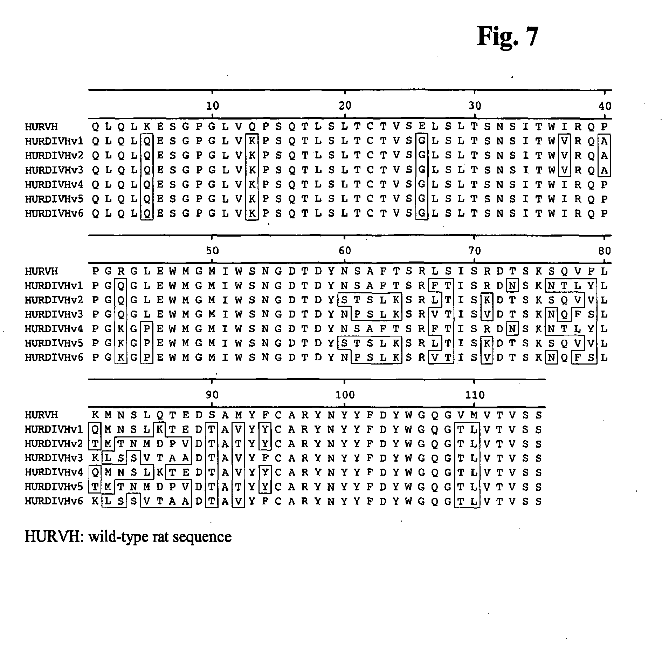

The invention further provides a method for the identification and/or manufacturing of an antibody against IL-1R with improved properties according to the invention. In this method, the polypeptide sequence of antibody 2D8 is modified by mutation, deletion or addition of amino acids in order to render the antibody less immunogenic when administered to humans. In general, up to about 50 amino acids are modified in the variable heavy chain and light chain to reduce immunogenicity. This modification is performed by comparison of the polypeptide sequence of 2D8 and the sequences of human antibodies provided by Kabat (loc. cit.) and/or the identification and elimination of T cell epitopes in the variable chains. Examples for such modifications are shown in Figs. 7-10. Useful antibodies can be generated by one or more changes in the enframed amino acids of these figures. Preferably, about 20-50 amino acids are modified in the enframed regions of the variable chains.

The invention therefore provides a method for the manufacturing of an antibody according to the invention, characterized in that in the sequences of the variable regions of antibody 2D8 one or more amino acids is/are mutated, deleted or added to the respective enframed amino acid(s) shown in Figs. 7-10, an expression vector is manufactured containing a nucleic acid encoding said modified antibody variable regions and additional human constant regions, preferably as shown in Figs. 14 to 16, in a consecutive reading frame, expression is performed in a host cell and the recombinantly produced antibody light and heavy chains are assembled together to an antibody according to the invention.

The invention further provides nucleic acids encoding such antibodies, expression vectors containing said nucleic acids, and host cells containing such vectors for the recombinant production of such antibodies.

The invention further provides methods for the recombinant production of such antibodies.

The invention further provides the use of an antibody according to the invention for the preparation of a medicament for the treatment of an inflammatory disease, especially for the treatment rheumatoid arthritis or osteoarthritis, of a patient diagnosed as having rheumatoid arthritis (and therefore being in need of an such a therapy) or ostheoathritis with an effective amount of an antagonistic antibody against IL-1R according to the invention. The antibody is administered preferably in a pharmaceutical composition.

The invention further comprises the use of an antibody according to the invention for rheumatoid arthritis treatment and for the manufacture of a pharmaceutical composition according to the invention. In addition, the invention comprises a method for the manufacture of a pharmaceutical composition according to the invention.

The invention further comprises a pharmaceutical composition containing an antibody according to the invention with a pharmaceutically effective amount, optionally together with a buffer and/or further excipients useful for the formulation of antibodies for pharmaceutical purposes.

The invention further provides pharmaceutical compositions comprising such antibodies in a pharmaceutically acceptable carrier. In one embodiment, the pharmaceutical composition may be included in an article of manufacture or kit.

The invention further comprises a vector containing a nucleic acid according to the invention, capable of expressing said nucleic acid in a prokaryotic or eukaryotic host cell.

The invention further comprises a prokaryotic or eukaryotic host cell comprising a vector according to the invention.

The invention further comprises a method for the production of a recombinant human antibody according to the invention, characterized by expressing a nucleic acid according to the invention in a prokaryotic or eukaryotic host cell and recovering said antibody from said cell. The invention further comprises the antibody obtainable by such a recombinant method.

### Detailed Description of the Invention

The term "antibody" encompasses the various forms of antibodies including but not being limited to whole antibodies, antibody fragments, humanized antibodies, chimeric antibodies, T cell epitope depleted antibodies, and further genetically engineered antibodies as long as the characteristic properties according to the invention are retained.

"Antibody fragments" comprise a portion of a full length antibody, generally at least the antigen binding portion or the variable region thereof. Examples of antibody fragments include diabodies, single-chain antibody molecules, immunotoxins, and multispecific antibodies formed from antibody fragments. In addition, antibody fragments comprise single chain polypeptides having the characteristics of a VH chain, namely being able to assemble together with a VL chain or of a VL chain binding to IL-1R, namely being able to assemble together with a VH chain to a functional antigen binding pocket and thereby providing the property of inhibiting the binding of IL-1 to IL-1R.

The terms "monoclonal antibody" or "monoclonal antibody composition" as used herein refer to a preparation of antibody molecules of a single amino acid composition. The term "chimeric antibody" refers to a monoclonal antibody comprising a variable region, i.e., binding region, from rat and at least a portion of a constant region derived from a different source or species, usually prepared by recombinant DNA techniques. Chimeric antibodies comprising a rat variable region and a human constant region are especially preferred. Such rat/human chimeric antibodies are the product of expressed immunoglobulin genes comprising DNA segments encoding rat immunoglobulin variable regions and DNA segments encoding human immunoglobulin constant regions. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art. See, e.g., Morrison, S.L., et al., Proc. Natl. Acad Sci. USA 81 (1984) 6851-6855; US Patent Nos. 5,202,238 and 5,204,244.

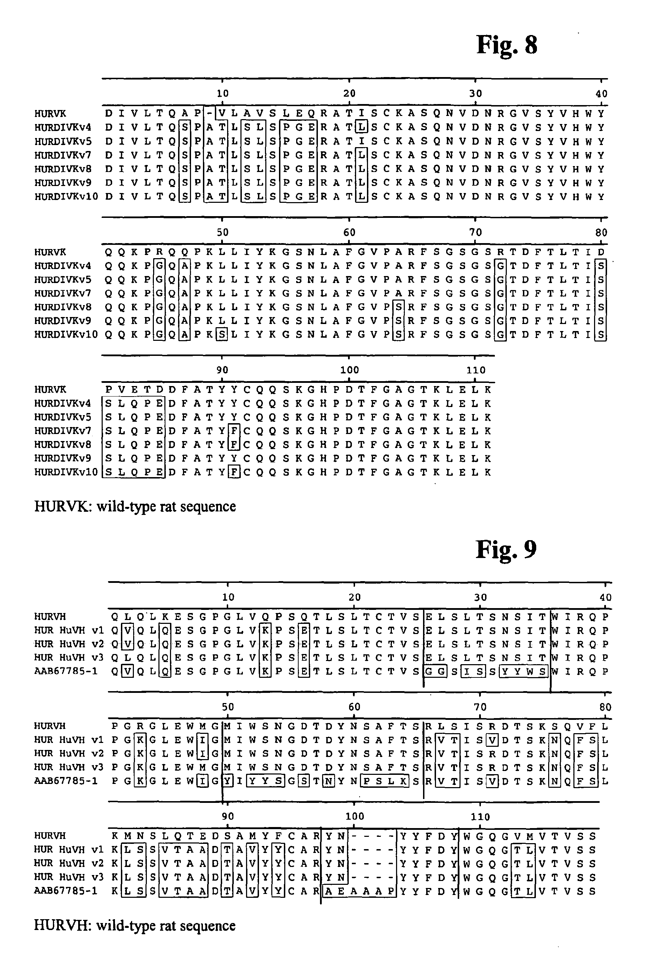

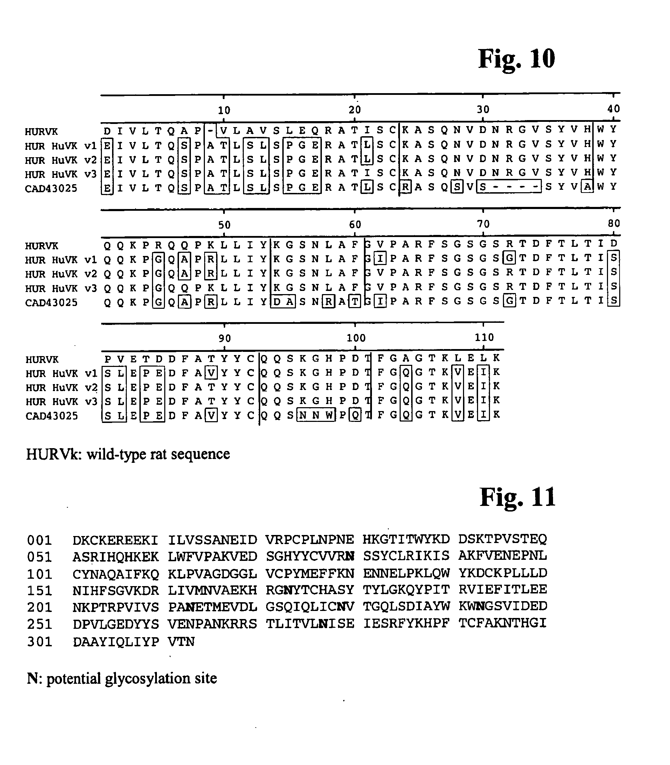

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a rat CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Particularly preferred CDRs correspond to those representing sequences recognizing the antigens noted above for chimeric and bifunctional antibodies. Examples for humanized antibodies according to the invention are shown in Figs. 9 and 10.

The term T cell epitope depleted antibody" refer to antibodies which were modified to remove or reduce immunogenicity by removing human T cell epitopes (peptide sequences within proteins with the capacity to bind to MHC Class II molecules).By this method interactions between amino acid side chains of the petide and specific binding pockets with the MHC class II binding groove are identified. The identified immunogenic regions are mutated to eliminate immunogenicity. Such methods are described in general in, e.g., WO 98/52976. Examples for T cell epitope depleted antibody variable regions useful and according to the invention are shown in Figs. 7 and 8.

As used herein, "binding" refers to antibodies binding to IL-1R with an affinity of about 300 pM or less, preferably about 200 pM or less (K_{D}), and more preferably of about 70-200 pM

The term "nucleic acid molecule", as used herein, is intended to include DNA molecules and RNA molecules. A nucleic acid molecule may be single-stranded or double-stranded, but preferably is double-stranded DNA.

The "variable region" (variable region of a light chain (VL), variable region of a heavy chain (VH)) as used herein denotes each of the pair of light and heavy chains which is involved directly in binding the antibody to the antigen. The domains of variable light and heavy chains have the same general structure and each domain comprises four framework (FR) regions whose sequences are widely conserved, connected by three "hypervariable regions" (or complementarity determining regions, CDRs). The framework regions adopt a β-sheet conformation and the CDRs may form loops connecting the β-sheet structure. The CDRs in each chain are held in their three-dimensional structure by the framework regions and form together with the CDRs from the other chain the antigen binding site. The antibody heavy and light chain CDR3 regions play a particularly important role in the binding specificity/affinity of the antibodies according to the invention and therefore provide a further object of the invention.

The terms "hypervariable region" or "antigen-binding portion of an antibody" when used herein refer to the amino acid residues of an antibody which are responsible for antigen-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. Especially, CDR3 of the heavy chain is the region which contributes most to antigen binding. CDR and FR regions are determined according to the standard definition of Kabat et al., Sequences of Proteins of Immunological Interest, 5th ed., Public Health Service, National Institutes of Health, Bethesda, MD (1991)) and/or those residues from a "hypervariable loop".

The term "binding to IL-1R" as used herein means the binding affinity of the antibody to IL-1R in an in vitro ELISA assay, preferably in a binding assay using biotinylated human IL-1R and streptavidin coated microtiter plates. Binding affinity to IL-1R can also be investigated by a Biacore assay (Biacore AB, Uppsala, Sweden). The affinity of the binding is defined by the terms kₒₙ (rate constant for the association of the antibody from the antibody/antigen complex), k_{off} (dissociation constant), and K_{D} (kₒₙ/k_{off}). The antibodies according to the invention show a K_{D} of 300 pM or less, preferably 200 pM or less, and more preferably 70-200 pM and inhibit the binding to IL-1R and bind preferably to IL-1R at the same position as does IL-1.

The term "IL-1R expressing cells" refers to such cells which are expressing IL-1 receptor. Such cells are, for example, human fibroblast cells like MRC5 cells.

The antibodies according to the invention show a binding to the same epitope of IL-1R as antibody 2D8 or are inhibited in binding to IL-1R due to steric hindrance of binding. Binding inhibition can be detected by an competitive assay using immobilized IL-1R and antibody 2D8. A signal reduction of 50% or more shows that the antibody competes with antibody 2D8.

The term "epitope" means a protein determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents. The epitope of IL-1R to which the antibodies according to the invention bind specifically is recognized by an antibody according to the invention on both native and denatured IL-1R. An antibody according to the invention binds to human IL-1R (glycosylated, soluble extracellular domain) about at least 50-fold, preferably at least 100-fold stronger than to deglycosylated IL-1R (after treatment with N-glycosidase F, measurement by Western blot). Therefore, an antibody according to the invention shows a significantly reduced affinity for the binding to deglycosylated IL-1R.

The antibodies according to the invention include, in addition; such antibodies having "conservative sequence modifications", nucleotide and amino acid sequence modifications which do not affect or alter the above-mentioned characteristics of the antibody according to the invention. Modifications can be introduced by standard techniques known in the art, such as site-directed mutagenesis and PCR-mediated mutagenesis. Conservative amino acid substitutions include ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g. glycine, asparagine, glutamine, serine, threonine, tyrosine, cysteine, tryptophan), nonpolar side chains (e.g., alanine; valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, a predicted nonessential amino acid residue in a human anti-IL-1R antibody can be preferably replaced with another amino acid residue from the same side chain family.

Amino acid substitutions can be performed by mutagenesis based upon molecular modeling as described by Riechmann, L., et al., Nature 332 (1988) 323-327 and Queen, C., et al., Proc. Natl. Acad. Sci. USA 86 (1989) 10029-10033.

The antibodies according to the invention preferably show serum half-lives of about at least 5 days in vivo (Cynomolgus or human), preferably 8 -15 days.

The antibodies according to the invention are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression, nucleic acids encoding light and heavy chains or fragments thereof are inserted into expression vectors by standard methods. Expression is performed in appropriate prokaryotic or eukaryotic host cells like CHO cells, NSO cells, SP2/0 cells, HEK293 cells, COS cells, yeast, or E.coli cells, and the antibody is recovered from the cells (supernatant or cells after lysis).

Recombinant production of antibodies is well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

The antibodies may be present in whole cells, in a cell lysate, or in a partially purified or substantially pure form. Purification is performed in order to eliminate other cellular components or other contaminants, e.g. other cellular nucleic acids or proteins, by standard techniques, including alkaline/SDS treatment, CsCl banding, column chromatography, agarose gel electrophoresis, and others well known in the art. See Ausubel, F., et al., ed. Current Protocols in Molecular Biology, Greene Publishing and Wiley Interscience, New York (1987).

Expression in NS0 cells is described by, e.g., Barnes, L.M., et al., Cytotechnology 32 (2000) 109-123; and Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; and Norderhaug, L., et al., J. Immunol. Methods 204 (1997) 77-87. A preferred transient expression system (HEK 293) is described by Schlaeger, E.-J., and Christensen, K., in Cytotechnology 30 (1999) 71-83 and by Schlaeger, E.-J., in J. Immunol. Methods 194 (1996) 191-199.

The control sequences that are suitable for prokaryotes, for example, include a promoter, optionally an operator sequence, and a ribosome binding site. Eukaryotic cells are known to utilize promoters, enhancers and polyadenylation signals.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The monoclonal antibodies are suitably separated from the culture medium by conventional immunoglobulin purification procedures such as, for example, protein A-Sepharose, hydroxylapatite chromatography, gel electrophoresis, dialysis, or affinity chromatography. DNA and RNA encoding the monoclonal antibodies is readily isolated and sequenced using conventional procedures. The hybridoma cells can serve as a source of such DNA and RNA. Once isolated, the DNA may be inserted into expression vectors, which are then transfected into host cells such as HEK 293 cells, CHO cells, or myeloma cells that do not otherwise produce immunoglobulin protein, to obtain the synthesis of recombinant monoclonal antibodies in the host cells.

Amino acid sequence variants of human IL-1R antibody are prepared by introducing appropriate nucleotide changes into the antibody DNA, or by peptide synthesis. Such modifications can be performed, however, only in a very limited range, e.g. as described above. For example, the modifications do not alter the abovementioned antibody characteristics such as the IgG isotype and epitope binding, but may improve the yield of the recombinant production, protein stability or facilitate the purification.

Any cysteine residue not involved in maintaining the proper conformation of the anti- IL-1R antibody also may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

Another type of amino acid variant of the antibody alters the original glycosylation pattern of the antibody. By altering is meant deleting one or more carbohydrate moieties found in the antibody, and/or adding one or more glycosylation sites that are not present in the antibody. Glycosylation of antibodies is typically N-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. Addition of glycosylation sites to the antibody is conveniently accomplished by altering the amino acid sequence such that it contains one or more of the above-described tripeptide sequences (for N-linked glycosylation sites).

Nucleic acid molecules encoding amino acid sequence variants of anti-IL-1R antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of humanized or a T cell epitope depleted anti-IL-1R antibody.

Another type of covalent modification involves chemically or enzymatically coupling glycosides to the antibody. These procedures are advantageous in that they do not require- production of the antibody in a host cell that has glycosylation capabilities for N- or O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330, and in Aplin, J.D., and Wriston, J.C. Jr., CRC Crit. Rev. Biochem. 4 (1981) 259-306.

Removal of any carbohydrate moieties present on the antibody may be accomplished chemically or enzymatically. Chemical deglycosylation requires exposure of the antibody to the compound trifluoromethanesulfonic acid, or an equivalent compound. This treatment results in the cleavage of most or all sugars except the linking sugar (N-acetylglucosamine or N- acetylgalactosamine), while leaving the antibody intact. Chemical deglycosylation is described by Sojahr, H.T., and Bahl, O.P., Arch. Biochem. Biophys. 259 (1987) 52-57 and by Edge, A.S., et al. Anal. Biochem. 118 (1981) 131-137. Enzymatic cleavage of carbohydrate moieties on antibodies can be achieved by the use of a variety of endo- and exo- glycosidases as described by Thotakura, N.R., and Bahl, O.P., Meth. Enzymol. 138 (1987) 350-359.

Another type of covalent modification of the antibody comprises linking the antibody to one of a variety of nonproteinaceous polymers, eg., polyethylene glycol, polypropylene glycol, or polyoxyalkylenes, in the manner set forth in US Patent Nos. 4,640,835; 4,496,689; 4,301, 144; 4,670,417; 4,791,192 or 4,179,337.

The invention preferably comprises a nucleic acid fragment encoding a polypeptide binding to IL-1R, whereby said polypeptide inhibits the binding of IL-1 to IL-1R, selected from the group consisting of a heavy chain consisting of a variable region (VH) of SEQ ID NO:1, and a light chain consisting of a variable region (VL) of SEQ ID NO:2.

The reconstructed heavy and light chain variable regions are combined with sequences of promoter, translation initiation, constant region, 3' untranslated, polyadenylation, and transcription termination to form expression vector constructs. The heavy and light chain expression constructs can be combined into a single vector, co-transfected, serially transfected, or separately transfected into host cells which are then fused to form a single host cell expressing both chains.

Accordingly, the invention provides a method for the production of a recombinant human antibody according to the invention, characterized by expressing a nucleic acid encoding a heavy chain consisting of a variable region (VH) of SEQ ID NO:1, and a light chain consisting of a variable region (VL) of SEQ ID NO:2, and of a human light chain constant region (CL) in a prokaryotic or eukaryotic host cell and recovering said antibody from said cell.

The invention further comprises the use of an antibody according to the invention for the diagnosis of IL-1R in vitro, preferably by an immunological assay determining the binding between IL-1R of a sample and the antibody according to the invention.

In another aspect, the present invention provides a composition, e.g. a pharmaceutical composition, containing one or a combination of monoclonal antibodies, or the antigen-binding portion thereof, of the present invention, formulated together with a pharmaceutically acceptable carrier.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g. by injection or infusion).

A "pharmaceutically acceptable salt" refers to a salt that retains the desired biological activity of the antibody and does not impart any undesired toxicological effects (see e.g. Berge, S.M., et al., J. Pharm. Sci. 66 (1977) 1-19). Such salts are included in the invention. Examples of such salts include acid addition salts and base addition salts. Acid addition salts include those derived from nontoxic inorganic acids, such as hydrochloric salts.

A composition of the present invention can be administered by a variety of methods known in the art. As will be appreciated by the skilled artisan, the route and/or mode of administration will vary depending upon the desired results.

To administer a compound of the invention by certain routes of administration, it may be necessary to coat the compound with, or co- administer the compound with, a material to prevent its inactivation. For example, the compound may be administered to a subject in an appropriate carrier, for example, liposomes, or a diluent. Pharmaceutically acceptable diluents include saline and aqueous buffer solutions.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

These compositions may also contain exipients such as preservatives, wetting agents, emulsifying agents and dispersing agents. Prevention of presence of microorganisms may be ensured both by sterilization procedures, or aseptic manufacturing conditions, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The composition must be sterile and fluid to the extent that the composition is deliverable by syringe. In addition to water, the carrier can be an isotonic buffered saline solution, ethanol, polyol (for example, glycerol, propylene glycol, and liquid polyetheylene glycol, and the like), and suitable mixtures thereof.

Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition. Long-term absorption of the injectable compositions can be brought about by including in the composition an agent which delays absorption, for example, aluminum monostearate or gelatin.

When the active compound is suitably protected, as described above, the compound may be orally administered, for example, with an inert diluent or an assimilable edible carrier.

The antibodies according to the invention can be used for the treatment of a patient suffering from rheumatoid arthritis and in the need of an such a therapy. Therefore, the invention comprises the use of an antibody according to the invention for the preparation of a medicament for the treatment of rheumatoid arthritis.

The invention further provides a method for the manufacture of a pharmaceutical composition comprising an effective amount of an antibody according to the invention together with a pharmaceutically acceptable carrier and the use of the antibody according to the invention for such a method.

### Description of the Figures

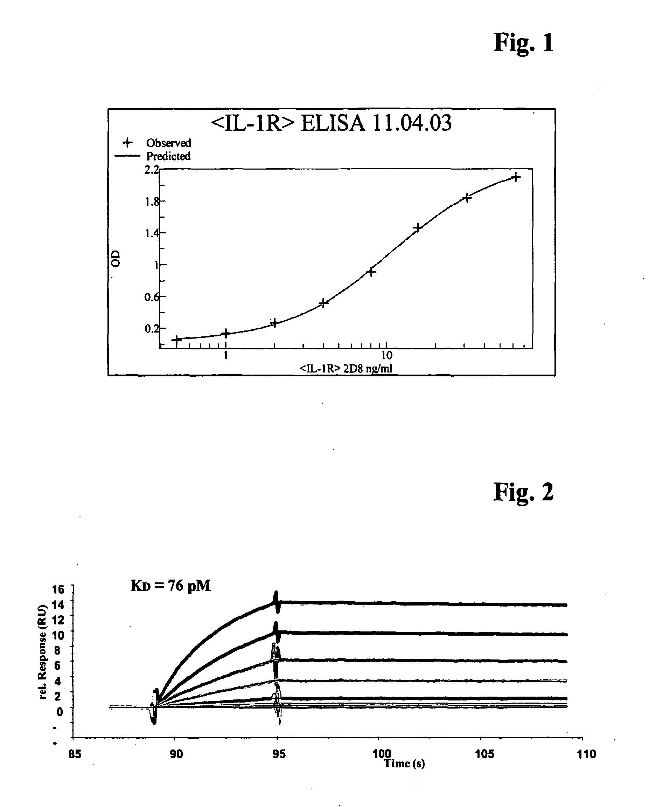

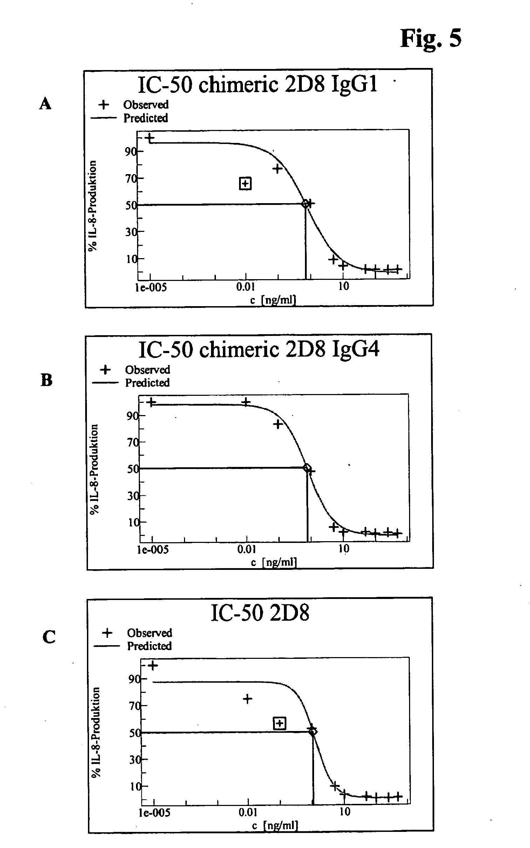

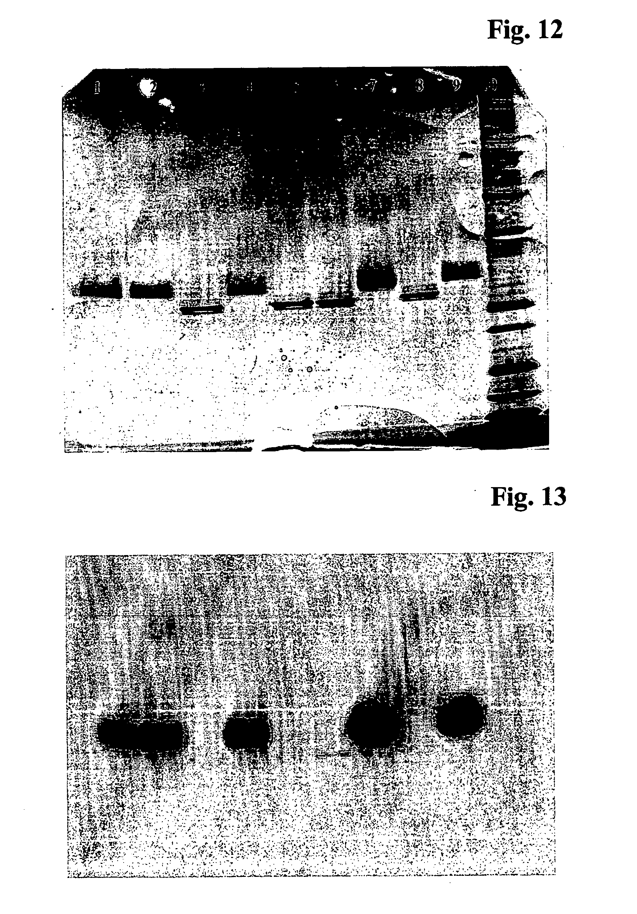

- **Figure 1**: ELISA binding assay for 2D8 antibodies
- **Figure 2**: Determination of the affinity of 2D8 antibodies to IL-1R

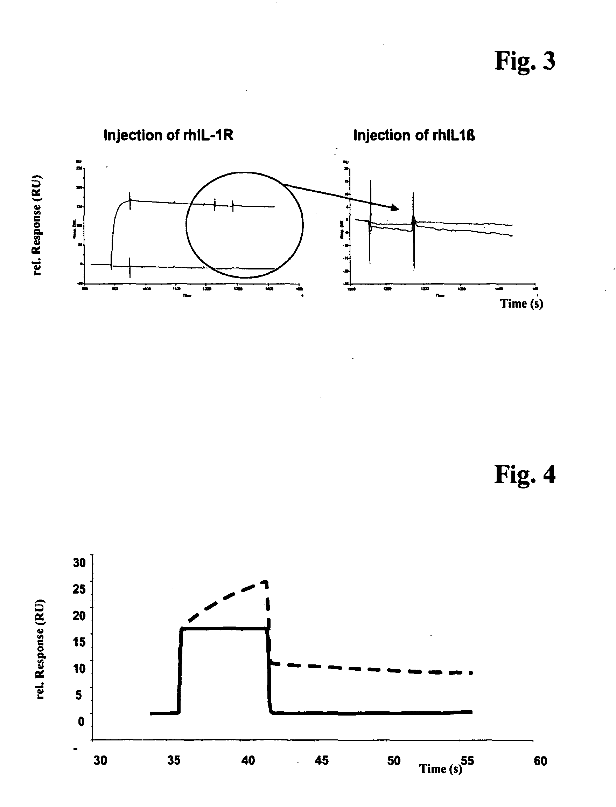

- **Figure 3**: Competition of 2D8 antibodies with the binding of the ligand IL-1 to IL-1R
- **Figure 4**: Inhibition of the ternary complex building hIL-1/ hIL-1R / hIL-1R AcP by 2D8 antibody
Upper line: binding of hIL-1/hIL-1R to hIL-1R Acp
Lower line: addition of antibody 2D8
- **Figure 5**: Inhibition of IL-1 induced production of IL-8 in MRC-5 cells

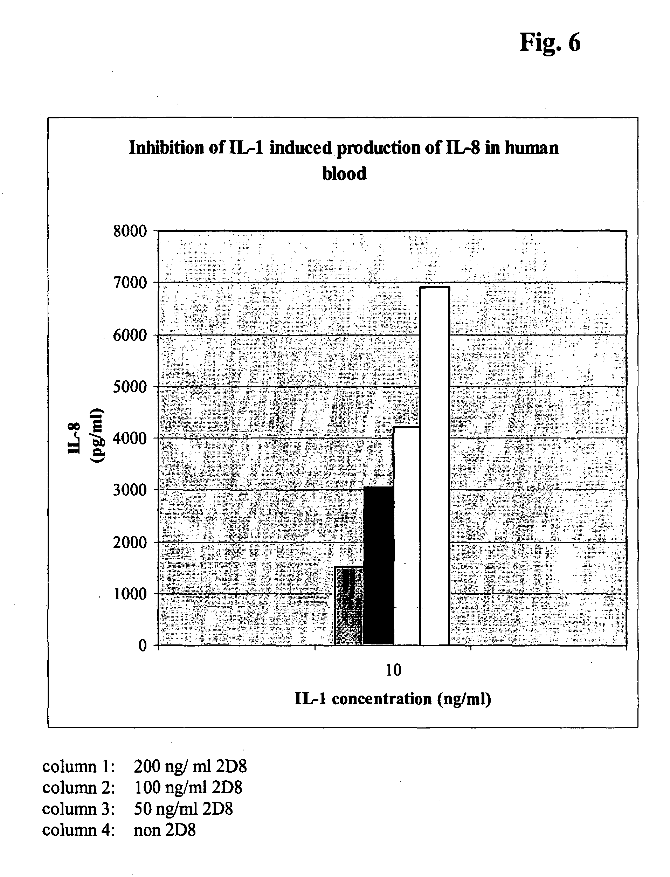

- **Figure 6**: Inhibition of IL-1 induced production of IL-8 in human blood
- **Figure 7**: Amino acid sequence of the variable regions of T cell epitope depleted heavy chains
- **Figure 8**: Amino acid sequence of the variable regions of T cell epitope depleted light chains
- **Figure 9**: Amino acid sequence of the variable regions of humanized heavy chains
- **Figure 10**: Amino acid sequence of the variable regions of humanized light chains
- **Figure 11**: Amino acid sequence of IL-1R
Bold N: potential glycosylation sites
- **Figure 12**: SDS PAGE analysis of glycosylated IL-1R and after treatment with glycosidases
- **Figure 13**: Western blot, 2D8 with glycosylated and deglycosylated IL-1R
- **Figure 14**: Constant region of a light chain
- **Figure 15**: Constant region of a heavy chain (IgG4)
- **Figure 16**: Constant region of a heavy chain (IgG1)

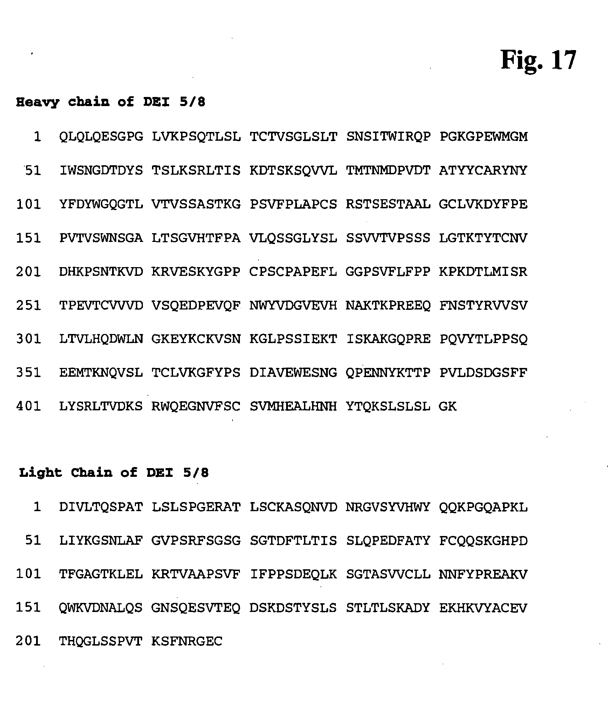

- **Figure 17**: Heavy and light chain of DEI 5/8

### Description of the Sequences

- SEQ ID NO:1: variable region of heavy chain of rat 2D8; aal-19 signal sequence, 20-134 variable region, 135-139 terminal fragment of rat origin
- SEQ ID NO:2: variable region of light chain of rat 2D8; aa 1-20 signal sequence, 21-129 variable region, 130-138 terminal fragment of rat origin
- SEQ ID NO:3: amino acid sequence of 2D8 chimeric H-chain (rat/human) (IgG1)
- SEQ ID NO:4: amino acid sequence of 2D8 chimeric H-chain (rat/human) (IgG4)
- SEQ ID NO:5: amino acid sequence of 2D8 chimeric L-chain (rat/human)
- SEQ ID NO:6: amino acid sequence of HURVH (Fig. 7 and Fig. 9)
- SEQ ID NO:7: amino acid sequence of HURDIVHv1 (Fig. 7)
- SEQ ID NO:8: amino acid sequence of HURDIVHv2 (Fig. 7)
- SEQ ID NO:9: amino acid sequence of HURDIVHv3 (Fig. 7)
- SEQ ID NO:10: amino acid sequence of HURDIVHv4 (Fig. 7)
- SEQ ID NO:11: amino acid sequence of HURDIVHv5 (Fig. 7)
- SEQ ID NO:12: amino acid sequence of HURDIVHv6 (Fig. 7)
- SEQ ID NO:13: amino acid sequence of HURVK (Fig. 8 and Fig. 10)
- SEQ ID NO:14: amino acid sequence of HURDIVKv4 (Fig. 8)
- SEQ ID NO:15: amino acid sequence of HURDIVKv5 (Fig. 8)
- SEQ ID NO:16: amino acid sequence of HURDIVKv7 (Fig. 8)
- SEQ ID NO:17: amino acid sequence of HURDIVKv8 (Fig. 8)
- SEQ ID NO:18: amino acid sequence of HURDIVKv9 (Fig. 8)
- SEQ ID NO:19: amino acid sequence of HURDIVKv10 (Fig. 8)
- SEQ ID NO:20: amino acid sequence of HUR HuVH v1 (Fig. 9)
- SEQ ID NO:21: amino acid sequence of HUR HuVH v2 (Fig. 9)
- SEQ ID NO:22: amino acid sequence of HUR HuVH v3 (Fig. 9)
- SEQ ID NO:23: amino acid sequence of AAB67785-1 (Fig. 9)
- SEQ ID NO:24: amino acid sequence of HUR HuVK v1 (Fig. 10)
- SEQ ID NO:25: amino acid sequence of HUR HuVK v2 (Fig. 10)
- SEQ ID NO:26: amino acid sequence of HUR HuVK v3 (Fig. 10)
- SEQ ID NO:27: amino acid sequence of CAD43025 (Fig. 10)
- SEQ ID NO:28: amino acid sequence of IL-1R (Fig. 11)
- SEQ ID NO:29: sequence of the constant region of the light chain (Fig. 14)
- SEQ ID NO:30: sequence of the heavy chain IgG4 (Fig. 15)
- SEQ ID NO:31: sequence of the constant region of IgG1 (Fig. 16)
- SEQ ID NO:32: sequence of the heavy chain of DEI 5/8 (Fig. 17)
- SEQ ID NO:33: sequence of the light chain of DEI 5/8 (Fig. 17)

### Examples

### Example 1

### Generation of rat monoclonal antibodies against h-IL-1R

### Culture of hybridomas

Generated rat monoclonal antibodies were cultured in RPMI 1640 and 10% Hyclone medium without serum ( BioWhittaker ) at 37°C and 5% CO₂.

### Immunization procedure and hybridoma development

5 Sprague Dawley rats were immunized with recombinant human IL-1R produced inSF9 insect cells. First immunization ( 100µg protein) is performed in complete Freunds' adjuvants intraperitonally. All other immunizations have been performed in incomplete Freunds adjuvants. Monoclonal antibodies were produced by fusing NSO cells with splenocytes from rat.

### Example 2

### IL-1R specific ELISA

Anti-IL-1R titers in sera of immunized mice or antibodies in culture supernatants were determined by antigen specific ELISA. Soluble biotinylated h-IL-1R at a concentration of 0.125 µg/ml in PBSBSA (PBS / 1% BSA) was coated 1 hour at RT on a shaker to 96 wells plates (precoated with streptavidin). Thereafter the wells were blocked with PBSBSA for 30 min. at RT. Sera were prediluted 1/100 in PBSBSA and serially diluted up to 1/6400. Supernatants were diluted by calculation in a range from 1/100 up to 1/10000 in PBSBSA. Diluted sera or supernatants were added to the wells and incubated for 2 h at RT on a shaker. Pre-tap serum or culture medium were used as negative controls. 125 ng/ml rat anti-human-IL-1R-antibody (clone 2D8) was used as positive control. Subsequently, plates were washed three times with PBS/0.05% Tween20 and incubated with horse radish peroxidase (HRP)-conjugated rabbit-anti-rat IgG (Bethyl Laboratories Inc.), diluted 1:18750 in PBSBSA for 1h at RT on a shaker. Wells were washed 4 times with PBS/0.05% Tween20 and assays were developed with freshly prepared TM Blue^{®} (Intergen Company) solution for 15-20 min. at RT in the dark on an shaker. Absorbance was measured at 370 nm with 492 nm as reference wavelength. Optionally, the color reaction can be stopped by addition of 20µl 1M H₂SO₄ to each well; in this case measurement were carried out at 450 nm with 690 nm as reference wavelength. The results are shown in Fig. 1.

### Example 3

### Determination of the binding properties of anti-hIL-1R antibody

The determination was performed on BIACORE^{®} 3000 using a CM5 chip. Coupling was performed as amine coupling. The buffer used was PBST (PBS + 0.05% Tween) pH 7.4, 25°C

### a) Determination of the affinity of anti-hIL-1R antibody to IL-LR

For affinity measurements an anti-Fcγ antibody (rabbit-anti-human) was coupled to the chip surface for presentation of the antibody against IL-1R. An anti-IL-IR antibody was bound to the anti-Fcγ antibody and recombinant human IL-1R extracellular domain was added in various concentrations in solution. Association was measured by an IL-1R injection of 60 seconds, dissociation was measured by washing the chip surface with buffer for 3 minutes. The affinity constant K_{D} (kₒₙ/k_{off}) for the antibodies are:

| | |
|---|---|
| antibody 2D8 | 100 pM |
| chimeric 2D8 (IgG1) | 93 pM |
| chimeric 2D8 (IgG4) | 105 pM |

Data range for all measurements of all antibodies is between 70-120 pM, therefore the affinities of these three antibodies are in the same range.

### b) Competition of the anti-hIL-1R antibody with the binding of the ligand IL-1 to IL-1R

For these measurements the same method as in a) was used. If hIL-1R is bound to anti-hIL-1R antibody 2D8, hIL-1 did not bind anymore to the receptor. This antibody blocks the binding site of hIL-1 on the receptor (Fig. 3).

### c) Inhibition of the ternary complex building hIL-1 / hIL-1R / hIL-1R AcP by the antibody anti-hIL-1R 2D8

For the detection of the ternary complex polyclonal anti-IgG antibody was coupled to the chip surface for presentation of the fusion protein Fc / hIL-1R AcP ( Swiss Prot Q9NPH3; R&D Sytems). Binding of the hIL-1 / hIL-1R complex in solution to the immobilized hIL-1R AcP was detected. Antibody anti-hIL-1R 2D8 inhibited the forming of this ternary complex hIL-1 / hIL-1R / hIL-1R AcP when incubated with hIL-1R (Fig. 4).

### Example 4

### a) Inhibition of IL-1 induced production of IL-8 in MRC-5 cells

The inhibition of the IL-1 induced production of IL-8 in human fibroblasts was determined in a cellular bioassay. Human embryonic lung fibroblasts MRC-5 were stimulated with h-IL1β and the production of IL-8 was determined in a one-step ELISA. Inhibition of the stimulation by addition of anti-h-IL-1R antibodies proves blocking function of the antibodies.

The Bioassays were run according the following assay procedure:

On Day 1 MRC-5 cells were seeded at a density of 2.5x10³ cells per well in 100µl culture medium (10% FBS) and incubated for 24 hours in an incubator.

On Day 2 culture medium was removed and samples were added in 50µl assay medium (1% FBS) (when testing purified antibodies) or Hybridoma-Medium SF (when testing Hybridoma supernatants). Optimal dilution of the Hybridoma supernatants was between 1:1000 and 1:1,000,000). Incubation was performed for 30 min. in an incubator. Subsequently h-IL-1β, 50µl/well (2x concentrated!) was added in assay-medium (1% FBS, or, if testing Hybridoma-supematants, 2% FBS), to a final FBS concentation ofl%. Further incubation was performed at 37°C for 7h and the supernatants (80µl) transferred in another 96-well plate.

### b) h-IL-8-ELISA

The assay (Miller, M.D., and Krangel, M.S., crit. Rev. Immunol. 12 (1992) 17-46) is performed according to the manufacturer's protocol (R&D Systems, USA) with the undiluted supernatants (protocol for blood and cerebrospinal liquid samples). The maximum of stimulation (controls without antibody) was approx. 750pg/ml IL-8. IC 50 for the inhibition of IL-8 secretion by the antibodies are shown in Fig. 5 and are:

| | |
|---|---|
| Antibody 2D8 | 7.2 pM (1.08 ng/ml) |
| Chimeric 2D8 (IgG1) | 4.7 pM (0.71 ng/ml) |
| Chimeric 2D8 (IgG4) | 5.1 pM (0.77 ng/ml) |

The range for all experiments with the antibodies according to the invention was 4.0 - 35.0 pM

A comparison of 2D8 with commercially available antibodies against II-1R in this MRC-5 bioassay is shown in Table 1:

**Table 1:**

| Manufacturer | Cat.# | Clone | Host | IgGTyp | Antigen | Neutralizing* | IC-50 |
|---|---|---|---|---|---|---|---|
| 2D8 | | | Rat | IgG2a | | yes | 1.08ng/ ml |
| Antigenix | MC260020 | R11 | Rat | IgG2a | CD121a | yes | > 100 ng/ml |
| Antigenix | MC261020 | R12 | Rat | IgG2a | CD121a | no | > 100 ng/ml |
| PBL Biomedical Laboratories | 21808-1 | RMH1R-1 | Rat | IgG2a | CD121a a | yes | 33ng/m l |
| PBL Biomedical Laboratories | 21809-1 | RMH1R-2 | Rat | IgG2a | CD121a | no | > 100 ng/ml |
| Cell Sciences | CM2028 | RMHL-1 | Rat | IgG2a | CD121a | yes | > 100 ng/ml |
| R&D Systems | MAB269 | 35730 | Mouse | IgG1 | s-IL-1RI | unknown | >100 ng/ml |
| BD Pharmingen | 551388 | HIL1R-M1 | Mouse | IgG1, kappa | CD121a | unknown | > 100 ng/ml |
| Biotrend | 0100-0242 | 49/20 | Mouse | IgG1 | s-IL-1RI | yes | > 100 ng/ml |

Based on the amino acid sequences of Figures 7-10 a panel of humanized and T cell antigen depleted antibodies are constructed by mutagenesis and investigated for IC50 by h-IL-8-ELISA. The results are shown in Table 2 in relation to antibody 2D8 (1.0).

### Explanation of abbreviations used:

- HUM:: Combination of sequences of Figs. 9 and 10
- DEI:: Combination of sequences of Figs. 7 and 8
- HUM2/2:: Combination of sequences HURHuVHv2 and HURHuVKv2
- DEII/8:: Combination of sequences HURDIVHv1 and HURDIVKv8

**Table 2:**

| **Antibody** | **Bioassay** | | |
|---|---|---|---|
| | 1^{st} experiment | 2^{nd} experiment | mean |
| 2D8 | - | - | 1.0 |
| DEI5/7 | 3.4 | 1.2 | 2.3 |
| DEI4/7 | 3.4 | 3.5 | 3.5 |
| DEI2/4 | 2.5 | 2.0 | 2.3 |
| DEI5/4 | 3.1 | 1.9 | 2.5 |
| DEI4/5 | 1.5 | 4.5 | 3.0 |
| DEI5/5 | 5.2 | 3.9 | 4.6 |
| HUM2/2 | 0.9 | 1.0 | 1.0 |
| HUM2/3 | 0.8 | 1.5 | 1.2 |
| DEII/8 | 3.2 | 0.7 | 2.0 |
| DEI2/8 | 3.3 | 1.4 | 2.4 |
| DEI2/9 | 2.7 | 2.8 | 2.8 |
| DEI4/9 | 5.2 | 4.1 | 4.7 |
| DEI5/8 | 1.7 | 2.2 | 2.0 |
| DEI5/9 | 1.6 | 2.8 | 2.2 |

### c) Determination of immunactivity by ELISA

The relative immunactivity of the antibodies as compared to 2D8 antibody was determined by ELISA. A soluble biotinylated human IL-1R was coated at room temperature for one hour to 96-well-plates (precoated with streptavidin). After blocking with BSA the antibody was diluted and added to the wells and incubated at room temperature for two hours. The plates were washed three times and incubated with horse-radish-peroxidase (POD)-conjugated rabbit-anti-rat-IgG at room temperature for one hour. After further washing the amount of bound antibody was determined by addition of TM-Blue^{R} and measurement of the absorbance at 370 nm.

The results are shown in Table 3.

**Table 3:**

| **Antibody** | **ELISA** | | |
|---|---|---|---|
| | 1^{st} experiment | 2^{nd} experiment | mean |
| 2D8 | - | - | 1.0 |
| DEI5/7 | 1.3 | 1.5 | 1.4 |
| DEI4/7 | 2.1 | 2.1 | 2.1 |
| DEI2/4 | 1.5 | 1.8 | 1.7 |
| DEI5/4 | 1.9 | 1.8 | 1.9 |
| DEI4/5 | 3.0 | 2.2 | 2.6 |
| DEI5/5 | 3.1 | 3.4 | 3.3 |
| HUM2/2 | 1.3 | 1.6 | 1.4 |
| HUM2/3 | 2.2 | 2.1 | 2.2 |
| DEI1/8 | 2.1 | 2.2 | 2.2 |
| DEI2/8 | 1.5 | 2.6 | 2.1 |
| DEI2/9 | 1.4 | 1.8 | 1.6 |
| DEI4/9 | 2.2 | 2.6 | 2.4 |
| DEI5/8 | 1.4 | 1.9 | 1.7 |
| DEI5/9 | 1.3 | 2.2 | 1.9 |

### Example 5

### Inhibition of IL-1 induced production of IL-8 in human blood

To assess the blocking capacity of antibody 2D8 to inhibit the IL-1-induced production of IL-8 in human blood, a bioassay was performed. Human blood collected with heparin (19U/ml) was stimulated with 10 ng/ml h-IL1β and the production of IL-8 was determined in one-step ELISA. Inhibition of the stimulation by addition of and-h-IL-1R antibodies proves blocking function of the antibodies in an human ex vivo assay. The bioassay was performed according to the description of Example 4 except that the incubation with IL-1 in the presence or absence of the antibody was performed at 37°C for 16h and 1:5 diluted samples were tested for IL-8 presence according to the manufacturer's protocol (R&D Systems, USA). IL-1 induced IL-8 production in human blood was inhibited by ~50% at a concentration of 100ng/ml 2D8 antibody (see Figure 6).

### Example 6

### Recombinant production of antibodies

Vectors for expression of a chimaeric antibody, consisting of human constant regions linked to murine variable regions, have been constructed. Two chimaeric heavy chain expression vectors have been constructed consisting of the anti-IL1R rat VH linked to human IgG1 and human IgG4 constant regions in the expression vector pSVgpt. A chimaeric light chain vector has been constructed consisting of anti-IL1R rat VK linked to human C Kappa in the expression vector pSVhyg. 5 flanking sequence including the leader signal peptide, leader intron and the murine immunoglobulin promoter, and 3 flanking sequence including the splice site and intron sequence was introduced in the chimaeric expression vectors.The heavy and light chain expression vectors were co-transfected into NSO cells (ECACC No 85110503, a non-immunoglobulin producing mouse myeloma) by electroporation. Transfected cell clones were screened for production of human antibody by ELISA for human IgG.

### Example 7

### Deglycosylation of IL-1R

5 µg of recombinant soluble human type 1 IL-1 receptor (IL-1R, amino acids sequence (see Fig. 11) purified from the supernatant of transformed SF9 insect cells was incubated with 3 mU neuraminidase (N) (Roche Diagnostics GmbH, DE; No. 269611), 0.3 U N-glycosidase F (Roche Diagnostics GmbH, DE; No. 1365185) and 0.15 mU U O-glycosidase (Roche Diagnostics GmbH, DE; No. 1347101), respectively, at 37 °C for 17 hours. After centrifugation the samples were analyzed by SDS-PAGE.

SDS-PAGE analysis (Fig. 12) demonstrated that the molecular weight of IL-1R (lane 1) was reduced by about 9 kDa upon incubation with N-glycosidase F (lane 3) while no significant changes of the molecular weight were observed upon incubation with neuraminidase (lane 2) and O-glycosidase (lane 4). The combination of N-glycosidase F with neuraminidase (lane 5), of N-glycosidase F and O-glycosidase (lane 6), or of all three (lane 8) did not result in any further cleavage of carbohydrate side chains. The combination of neuraminidase and O-glycosidase did not remove any carbohydrate side chains (lane 7).

The data demonstrate that IL-1R is glycosylated at one or more of the potential N-glycosylation sites and that the N-linked glycosylation can be completely removed by incubation with N-glycosidase F. No O-linked glycosylation was detected.

Western blot data for 2D8 antibody are shown in Fig. 13 (lanes as in Fig. 12).

### Example 8

### Determination of epitope

The binding region of an antibody on the antigen molecule (epitope) is determined by blotting and pepscan analysis.

For blotting analyses native or denaturated recombinant, soluble human IL-1R (rshIL-1R) samples were spotted directly on the membrane, incubated with anti-hIL1R antibodies and detected with anti-FC gamma-POD antibodies by chemiluminescense detection. In addition, rshIL-1R was deglycosylated with N-Glycosidase F and both glycosylated and deglycosylated rshIL-1R were separated under reducing denaturating conditions on SDS-PAGE. The proteins were blotted on PVDF-membranes, incubated with anti-hIL-1R antibodies and detected with anti-FCy-POD-antibodies by chemiluminescense detection.

Dot Blot Analysis showed that antibodies according to the invention specifically bound to rshIL-1R under native and denaturing conditions. Finally, western blot analysis showed that the antibodies according to the invention only recognizes glycosylated rshIL1R (native or denatured). Deglycosylated hIL1R could- not be detected in western blot analysis. Other glycosylated proteins (e.g. erythropoietin, carboxypeptidaseY) were not recognized by the antibodies according to the invention, so that the recognition of glycosylated rshIL-1R is highly specific.

In addition, biotinylated peptides (20 amino acids) representing the extra cellular domain of rshIL1R were synthesized for use in anti-IL-1R /rshIL-1R pepscan analysis. The peptides overlap by 10 amino acids and contain an inert N-terminally biotinylated spacer. The biotinylated peptides were coupled to streptavidine coated micro titer plates, incubated with anti-hIL1R antibodies and detected with anti-FCγ -antibody. Bound peptides represent the recognized sequence epitope of IL-1R.

### Example 9

### Binding of 2D8 and DEI5/8 to II-1R in the presence of IL-1R antagonist

The protein-protein interaction of anti-IL-1R antibodies with IL-1R is analyzed by surface plasmon resonance using a Biacore 3000 System. Association rates are determined by an injection of two minutes duration; dissociation rates are measured by a washing step of three minutes duration. Negative control data are subtracted from original curves for correction of system intrinsic baseline drift and for noise signal reduction. Two assay formats with the antibody and IL-1R immobilized to the chip surface are used for this study.
a) Anti-IL-1R antibody 2D8 is covalently immobilized on the chip surface (CM5) by amine coupling. IL-1R is injected in a concentration of 10 nM under mass transfer conditions in order to determine the maximum signal which serves as a reference in the inhibition studies. Inhibition of IL-1R (10nM) binding is measured by preincubation (in HBS-P buffer for at least 20 min at room temperature) of IL-1R with increasing concentrations (0.78-100 nM) of IL-1R antagonist, IL-1beta or DEI5/8. Subsequently, the samples are injected into the flow cells and interaction with immobilized 2D8 antibody is determined.
b) IL-1beta is covalently immobilized on the chip surface (CM5) by amine coupling. IL-1R is injected in a concentration of 10 nM under mass transfer conditions in order to determine the maximum signal which served as reference value. Inhibition of rshIL-1R (10nM) binding is measured by pre-incubation of IL-1R with increasing concentrations (0.78 -100 nM) of IL-1R antagonist, rhIL-1beta or DEI5/8. Subsequently, the samples are injected into the flow cells and interaction with immobilized rhIL-1beta is determined.

IL-1R antagonist shows in the first assay no inhibition of IL-1R binding at a concentration of 100 µM. IL-1beta shows half maximal inhibition (IC50) at 8 nM, DEI5/8 at 2 nM. In the second assay IL-1R antagonist shows an inhibitory activity (IC50) of 4 nM, DE15/8 of 2nM and 2D8 of 2 nM.

Since IL-1R antagonist do not inhibit binding of IL-1R to 2D8 in a concentration of up to 100 µM, it is concluded that 2D8 and IL-1R antagonist do not bind to IL-1R in a competitive manner. 2D8 induces an immediate conformation change of IL-1R upon binding. This conformation change of IL-1R does no longer allow the binding of IL-1R antagonist to the receptor. 2D8 and DEI5/8 show allosteric inhibition versus IL-1R antagonist in binding to the receptor. IL-1beta inhibits IL-1R binding to 2D8, indicating a competitive binding of IL-1beta and 2D8 to IL-1R.

### List of References

Angal, S., et al., Mol. Immunol. 30 (1993) 105-108
Aplin, J.D., and Wriston, J.C. Jr., CRC Crit. Rev. Biochem. 4 (1981) 259-306
Arend W.P., J. Clin. Invest. 88 (1991) 1445-1451
Ausubel, F., et al., ed. Current Protocols in Molecular Biology, Greene Publishing and Wiley Interscience, New York (1987)
Barnes, L.M., et al., Biotech. Bioeng. 73 (2001) 261-270
Barnes, L.M., et al., Cytotechnology 32 (2000) 109-123
Berge, S.M., et al., J. Pharm. Sci. 66 (1977) 1-19
Carter, P., et al., Proc. Natl. Acad. Sci. USA 89 (1992) 4285-4289
Durocher, Y., et al., Nucl. Acids. Res. 30 (2002) E9
Edge, A.S., et al. Anal. Biochem. 118 (1981) 131-137
Geisse, S., et al., Protein Expr. Purif. 8 (1996) 271-282
Johnson, G., and Wu, T.T., Nucleic Acids Res. 28 (2000) 214-218
Kabat et al., Sequences of Proteins of Immunological Interest, 5th ed., Public Health Service, National Institutes of Health, Bethesda, MD (1991)
Kaufman, R.J., Mol. Biotechnol. 16 (2000) 151-161
Makrides, S.C., Protein Expr. Purif. 17 (1999) 183-202
Miller, M.D., and Krangel, M.S., crit. Rev. Immunol. 12 (1992) 17-46
Morrison, S.L., et al., Proc. Natl. Acad Sci. USA 81 (1984) 6851-6855
Neuberger, M.S., et al., Nature 314 (1985) 268-270
Norderhaug, L., et al., J. Immunol. Methods 204 (1997) 77-87
Orlandi, R., et al., Proc. Natl. Acad. Sci. USA 86 (1989) 3833-3837
Queen, C., et al., Proc. Natl. Acad. Sci. USA 86 (1989) 10029-10033
Riechmann, L., et al., Nature 332 (1988) 323-327
Schlaeger, E.-J., and Christensen, K., Cytotechnology 30 (1999) 71-83
Schlaeger, E.-J., J. Immunol. Methods 194 (1996) 191-199
Sojahr, H.T., and Bahl, O.P., Arch. Biochem. Biophys. 259 (1987) 52-57
Thotakura, N.R., and Bahl, O.P., Meth. Enzymol. 138 (1987) 350-359
US Patent No. 4,179,337
US Patent No. 4,496,689
US Patent No. 4,640,835
US Patent No. 4,670,417
US Patent No. 4,791,192
US Patent No. 5,202,238
US Patent No. 5,204,244
US Patent No. 6,511,665
Werner, R.G., Drug Res. 48 (1998) 870-880
WO 87/05330
WO 98/08097
WO 98/52976

## Claims

1. Antibody binding to human IL-1R and inhibiting the binding of human IL-1 to IL-1R, **characterized in that** said antibody is obtainable from hybridoma cell line MAK<h-IL-IRI>2D8 DSM ACC 2601 or is a chimeric, humanized or T cell epitope depleted antibody variant or a fragment of said antibody showing an IC50 value of 35 pM or lower for the inhibition of IL-1 mediated secretion of Il-8 and IL-6 in human fibroblast cells MRC5 ,ATCC CCL 171.

2. Antibody according to claim 1, **characterized in that** said antibody is produced by hybridoma cell line MAK<h-IL-1RI>2D8 DSM ACC 2601.

3. Antibody according to claim 1 or 2, **characterized in that** said antibody is of human IgG4 isotype or is of human IgG1 isotype, modified in the hinge region at aa 220-240 between CH1 and CH2 and/or the second inter-domain region of aa 330 between CH2 and CH3 (numbering according to Kabat).

4. Antibody according to claim 1, **characterized in that** said antibody shows variable and constant regions selected from Seq ID 6 to Seq ID 27 and Seq ID 29 - 31*.*

5. Antibody according to claims 1 to 4 **characterized by** comprising as complementarity determining regions, CDRs, the following sequences:
a) in the heavy chain as CDRs CDR1, aa 45-54, CDR2, aa 69-84, and CDR3, aa 117-123, of SEQ ID NO:1, and
b) in the light chain as CDRs CDR1, aa 43-57, CDR2, aa 73-79, and CDR3, aa 112-120, of SEQ ID NO:2.

6. A pharmaceutical composition containing an antibody according to claims 1 to 5 in a pharmaceutically effective amount.

7. Hybridoma cell lines MAK<h-IL-1RI>2D8 DSM ACC 2601.

8. Use of an antibody according to claims 1 to 5 for the preparation of a medicament for the treatment of an inflammatory disease.

9. Use according to claim 8, wherein the inflammatory disease is rheumatoid arthritis or osteoarthritis.

10. Method for the manufacturing of an antibody according to claims 1 to 5, **characterized in that** in the sequences of SEQ ID NO: 1 and SEQ ID NO: 2 of the variable regions of antibody 2D8 DSM ACC 2601 one or more amino acids is/are mutated, deleted or added to the respective enframed amino acid(s) shown in SEQ ID NO: 6 to SEQ ID NO: 27 in Figs. 7-10, an expression vector is manufactured containing a nucleic acid encoding said modified antibody variable regions and additional human constant regions, as shown in SEQ ID NO: 29 to SEQ ID NO: 31 in Figs. 14 to 16, in a consecutive reading frame, expression is performed in a host cell and the recombinantly produced antibody light and heavy chains are assembled together to an antibody according to the invention.

## Patentansprüche

1. Antikörper, der an humanen IL-1R bindet und die Bindung von humanem IL-1 an IL-1R inhibiert, **dadurch gekennzeichnet, dass** der Antikörper aus der Hybridomzelllinie MAK<h-IL-1RI>2D8 DSM ACC 2601 erhältlich ist oder eine chimäre, humanisierte oder T-Zell-Epitop-depletierte Antikörper-Variante oder ein Fragment des Antikörpers ist, der einen IC50-Wert von 35 pM oder niedriger für die Inhibierung von IL-1 vermittelter Sekretion von IL-8 und IL-6 in den humanen Fibroblastenzellen MRCSATCC CCL 171 aufweist.

2. Antikörper nach Anspruch 1, **dadurch gekennzeichnet, dass** der Antikörper durch die Hybridomzelllinie MAK<h-IL-1RI>2D8 DSM ACC 2601 hergestellt wird.

3. Antikörper nach Anspruch 1 oder 2, **dadurch gekennzeichnet, dass** der Antikörper vom humanen IgG4-Isotyp ist oder vom humanen IgG1-Isotyp ist, der in der Gelenkregion bei AS 220-240 zwischen CH1 und CH2 und/oder in der zweiten Zwischen-Domänen-Region mit AS 330 zwischen CH2 und CH3 (Nummerierung entsprechend Kabat) modifiziert ist.

4. Antikörper nach Anspruch 1, **dadurch gekennzeichnet, dass** der Antikörper variable und konstante Regionen, ausgewählt aus SEQ ID NO: 6 bis SEQ ID NO: 27 und SEQ ID NO: 29-31, aufweist.

5. Antikörper nach den Ansprüchen 1 bis 4, **dadurch gekennzeichnet, dass** er als komplementaritätsbestimmende Regionen (complementarity determining regions), CDRs, folgende Sequenzen umfasst:
a) als CDRs in der schweren Kette CDR1, AS 45-54, CDR2, AS 69-84, und CDR3, AS 117-123, der SEQ ID NO: 1, und
b) als CDRs in der leichten Kette CDR1, AS 43-57, CDR2, AS 73-79, und CDR3, AS 112-120, der SEQ ID NO: 2.

6. Pharmazeutische Zusammensetzung, die einen Antikörper nach den Ansprüchen 1 bis 5 in einer pharmazeutisch wirksamen Menge enthält.

7. Hybridomzelllinien MAK<h-IL-1RI>2D8 DSM ACC 2601.

8. Verwendung eines Antikörpers nach den Ansprüchen 1 bis 5 für die Herstellung eines Medikamentes zur Behandlung einer Entzündungskrankheit.

9. Verwendung nach Anspruch 8, wobei die Entzündungskrankheit rheumatoide Arthritis oder Osteoarthritis ist.

10. Verfahren zur Herstellung eines Antikörpers nach den Ansprüchen 1 bis 5, **dadurch gekennzeichnet, dass** in den Sequenzen der SEQ ID NO: 1 und SEQ ID NO: 2 der variablen Regionen des Antikörpers 2D8 DSM ACC 2601 eine oder mehrere Aminosäure(n) mutiert, deletiert oder an die entsprechende(n) eingerahmte(n) Aminosäure(n), die in SEQ ID NO: 6 bis SEQ ID NO:27 in den Figuren 7-10 gezeigt ist/sind, hinzugefügt ist/sind, ein Expressionsvektor hergestellt wird, der eine Nukleinsäure enthält, die für die modifizierten variablen Antikörper-Regionen und zusätzlichen humanen konstanten Regionen kodiert, wie in SEQ ID NO:29 bis SEQ ID NO:31 in den Figuren 14 bis 16 gezeigt, in einem fortlaufenden Leserahmen, die Expression in einer Wirtszelle durchgeführt wird und die rekombinant hergestellten leichten und schweren Antikörper-Ketten zu einem Antikörper entsprechend der Erfindung zusammengesetzt werden.

## Revendications

1. Anticorps se liant à l'IL-1R humain et inhibant la liaison de l'IL-1 humaine à l'IL-1R, **caractérisé en ce que** ledit anticorps peut être obtenu à partir de la lignée cellulaire d'hybridome MAK<h-IL-1RI>2D8 DSM ACC 2601 ou est une variante d'anticorps chimère, humanisée ou dépourvue d'épitopes des lymphocytes T ou un fragment dudit anticorps présentant une valeur de CI₅₀ d'au plus 35 pM pour l'inhibition de la sécrétion d'IL-8 et d'IL-6 médiée par l'IL-1 dans des cellules fibroblastes humaines MRC5 ATCC CCL 171.

2. Anticorps selon la revendication 1, **caractérisé en ce que** ledit anticorps est produit par la lignée cellulaire d'hybridome MAK<h-IL-1RI>2D8 DSM ACC 2601.

3. Anticorps selon la revendication 1 ou 2, **caractérisé en ce que** ledit anticorps est de l'isotype IgG4 humain ou est de l'isotype IgG 1 humain, modifié dans la région charnière au niveau des aa 220-240 entre CH1 et CH2 et/ou dans la deuxième région interdomaine de aa 330 entre CH2 et CH3 (numérotation de Kabat).

4. Anticorps selon la revendication 1, **caractérisé en ce que** ledit anticorps présente des régions variables et constantes choisies parmi SEQ ID NO : 6 à SEQ ID NO : 27 et SEQ ID NO : 29-31.

5. Anticorps selon les revendications 1 à 4, **caractérisé en ce qu'**il comprend comme régions déterminant la complémentarité, ou CDR, les séquences suivantes:
a) comme CDR dans la chaîne lourde CDR1, aa 45-54, CDR2, aa 69-84, et CDR3, aa 117-123, de SEQ ID NO: 1, et
b) comme CDR dans la chaîne légère CDR1, aa 43-57, CDR2, aa 73-79, et CDR3, aa 112-120, de SEQ ID NO: 2.

6. Composition pharmaceutique contenant un anticorps selon les revendications 1 à 5 en une quantité pharmaceutiquement efficace.

7. Lignées cellulaires d'hybridomes MAK<h-IL-1RI>2D8 DSM ACC 2601.

8. Utilisation d'un anticorps selon les revendications 1 à 5 pour la préparation d'un médicament destiné au traitement d'une maladie inflammatoire.

9. Utilisation selon la revendication 8, dans laquelle la maladie inflammatoire est la polyarthrite rhumatoïde ou l'arthrose.

10. Procédé de fabrication d'un anticorps selon les revendications 1 à 5, **caractérisé en ce que**, dans les séquences de SEQ ID NO: 1 et SEQ ID NO: 2 des régions variables de l'anticorps 2D8 DSM ACC 2601, on procède à la mutation, la délétion ou l'addition d'un ou plusieurs aminoacides pour obtenir les aminoacides respectifs encadrés présentés dans SEQ ID NO: 6 à SEQ ID NO: 27 sur les figures 7-10, on produit un vecteur d'expression contenant un acide nucléique codant pour lesdites régions variables de l'anticorps modifié et en plus des régions constantes humaines, de la manière indiquée dans SEQ ID NO: 29 à SEQ ID NO: 31 sur les figures 14 à 16; dans un cadre de lecture consécutif, on effectue l'expression dans une cellule hôte et on assemble entre elles les chaînes lourdes et légères de l'anticorps produites par recombinaison pour obtenir un anticorps selon l'invention.


### SEQUENCE LISTING

<110> F. Hoffmann-La Roche AG
<120> Antibodies against interleukin-1 receptor and uses thereof
<130> 21842
<150> US 60/501681
   <151> 2003-09-10
<150> EP 03029659
   <151> 2003-12-23
<160> 33
<170> PatentIn version 3.2
<210> 1
   <211> 139
   <212> PRT
   <213> rat
<400> 1
   Met Ala Val Leu Gly Leu Phe Phe Cys Leu Leu Ile Phe Pro Ser Cys
   1               5                   10                  15
   Val Leu Ser Gln Leu Gln Leu Lys Glu Ser Gly Pro Gly Leu Val Gln
               20                  25                  30
   Pro Ser Gln Thr Leu Ser Leu Thr Cys Thr Val Ser Glu Leu Ser Leu
           35                  40                  45
   Thr Ser Asn Ser Ile Thr Trp Ile Arg Gln Pro Pro Gly Arg Gly Leu
       50                  55                  60
   Glu Trp Met Gly Met Ile Trp Ser Asn Gly Asp Thr Asp Tyr Asn Ser
   65                  70                  75                  80
   Ala Phe Thr Ser Arg Leu Ser Ile Ser Arg Asp Thr Ser Lys Ser Gln
                   85                  90                  95
   Val Phe Leu Lys Met Asn Ser Leu Gln Thr Glu Asp Ser Ala Met Tyr
               100                 105                 110
   Phe Cys Ala Arg Tyr Asn Tyr Tyr Phe Asp Tyr Trp Gly Gln Gly Val
           115                 120                 125
   Met Val Thr Val Ser Ser Ala Glu Thr Thr Ala
       130                 135
<210> 2
   <211> 138
   <212> PRT
   <213> rat
<400> 2
   Met Glu Thr Asp Thr Leu Leu Leu Trp Val Leu Leu Leu Trp Val Pro
   1               5                   10                  15
   Gly Ser Thr Gly Asp Ile Val Leu Thr Gln Ala Pro Val Leu Ala Val
               20                  25                  30
   Ser Leu Glu Gln Arg Ala Thr Ile Ser Cys Lys Ala Ser Gln Asn Val
           35                  40                  45
   Asp Asn Arg Gly Val Ser Tyr Val His Trp Tyr Gln Gln Lys Pro Arg
       50                  55                  60
   Gln Gln Pro Lys Leu Leu Ile Tyr Lys Gly Ser Asn Leu Ala Phe Gly
   65                  70                  75                  80
   Val Pro Ala Arg Phe Ser Gly Ser Gly Ser Arg Thr Asp Phe Thr Leu
                   85                  90                  95
   Thr Ile Asp Pro Val Glu Thr Asp Asp Phe Ala Thr Tyr Tyr Cys Gln
               100                 105                 110
   Gln Ser Lys Gly His Pro Asp Thr Phe Gly Ala Gly Thr Lys Leu Glu
           115                 120                 125
   Leu Lys Arg Ala Asp Ala Ala Pro Thr Val
       130                 135
<210> 3
   <211> 445
   <212> PRT
   <213> chimeric (rat/human)
<400> 3
   Gln Leu Gln Leu Lys Glu Ser Gly Pro Gly Leu Val Gln Pro Ser Gln
   1               5                   10                  15
   Thr Leu Ser Leu Thr Cys Thr Val Ser Glu Leu Ser Leu Thr Ser Asn
               20                  25                  30
   Ser Ile Thr Trp Ile Arg Gln Pro Pro Gly Arg Gly Leu Glu Trp Met
           35                  40                  45
   Gly Met Ile Trp Ser Asn Gly Asp Thr Asp Tyr Asn Ser Ala Phe Thr
       50                  55                  60
   Ser Arg Leu Ser Ile Ser Arg Asp Thr Ser Lys Ser Gln Val Phe Leu
   65                  70                  75                  80
   Lys Met Asn Ser Leu Gln Thr Glu Asp Ser Ala Met Tyr Phe Cys Ala
                   85                  90                  95
   Arg Tyr Asn Tyr Tyr Phe Asp Tyr Trp Gly Gln Gly Val Met Val Thr
               100                 105                 110
   Val Ser Ser Ala Ser Thr Lys Gly Pro Ser Val Phe Pro Leu Ala Pro
           115                 120                 125
   Ser Ser Lys Ser Thr Ser Gly Gly Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Gln Thr Tyr Ile Cys Asn Val Asn His Lys Pro Ser Asn Thr Lys
           195                 200                 205
   Val Asp Lys Lys Val Glu Pro Lys Ser Cys Asp Lys Thr His Thr Cys
   210 215 220
   Pro Pro Cys Pro Ala Pro Glu Leu Leu Gly Gly Pro Ser Val Phe Leu
   225 230 235 240
   Phe Pro Pro Lys Pro Lys Asp Thr Leu Met Ile Ser Arg Thr Pro Glu
   245 250 255
   Val Thr Cys Val Val Val Asp Val Ser His Glu Asp Pro Glu Val Lys
   260 265 270
   Phe Asn Trp Tyr Val Asp Gly Val Glu Val His Asn Ala Lys Thr Lys
   275 280 285
   Pro Arg Glu Glu Gln Tyr Asn Ser Thr Tyr Arg Val Val Ser Val Leu
   290 295 300
   Thr Val Leu His Gln Asp Trp Leu Asn Gly Lys Glu Tyr Lys Cys Lys
   305 310 315 320
   Val Ser Asn Lys Ala Leu Pro Ala Pro Ile Glu Lys Thr Ile Ser Lys
   325 330 335
   Ala Lys Gly Gln Pro Arg Glu Pro Gln Val Tyr Thr Leu Pro Pro Ser
   340 345 350
   Arg Asp Glu Leu Thr Lys Asn Gln Val Ser Leu Thr Cys Leu Val Lys
   355 360 365
   Gly Phe Tyr Pro Ser Asp Ile Ala Val Glu Trp Glu Ser Asn Gly Gln
   370 375 380
   Pro Glu Asn Asn Tyr Lys Thr Thr Pro Pro Val Leu Asp Ser Asp Gly
   385 390 395 400
   Ser Phe Phe Leu Tyr Ser Lys Leu Thr Val Asp Lys Ser Arg Trp Gln
   405 410 415
   Gln Gly Asn Val Phe Ser Cys Ser Val Met His Glu Ala Leu His Asn
   420 425 430
   His Tyr Thr Gln Lys Ser Leu Ser Leu Ser Pro Gly Lys
           435                 440                 445
<210> 4
   <211> 442
   <212> PRT
   <213> chimeric (rat/human)
<400> 4
   Gln Leu Gln Leu Lys Glu Ser Gly Pro Gly Leu Val Gln Pro Ser Gln
   1               5                   10                  15
   Thr Leu Ser Leu Thr Cys Thr Val Ser Glu Leu Ser Leu Thr Ser Asn
               20                  25                  30
   Ser Ile Thr Trp Ile Arg Gln Pro Pro Gly Arg Gly Leu Glu Trp Met
           35                  40                  45
   Gly Met Ile Trp Ser Asn Gly Asp Thr Asp Tyr Asn Ser Ala Phe Thr
       50                  55                  60
   Ser Arg Leu Ser Ile Ser Arg Asp Thr Ser Lys Ser Gln Val Phe Leu
   65                  70                  75                  80
   Lys Met Asn Ser Leu Gln Thr Glu Asp Ser Ala Met Tyr Phe Cys Ala
                   85                  90                  95
   Arg Tyr Asn Tyr Tyr Phe Asp Tyr Trp Gly Gln Gly Val Met Val Thr
               100                 105                 110
   Val Ser Ser Ala Ser Thr Lys Gly Pro Ser Val Phe Pro Leu Ala Pro
           115                 120                 125
   Cys Ser Arg Ser Thr Ser Glu Ser Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Lys Thr Tyr Thr Cys Asn Val Asp His Lys Pro Ser Asn Thr Lys
           195                 200                 205
   Val Asp Lys Arg Val Glu Ser Lys Tyr Gly Pro Pro Cys Pro Ser Cys
       210                 215                 220
   Pro Ala Pro Glu Phe Leu Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser Gln Glu Asp Pro Glu Val Gln Phe Asn Trp
               260                 265                 270
   Tyr Val Asp Gly Val Glu Val His Asn Ala Lys Thr Lys Pro Arg Glu
           275                 280                 285
   Glu Gln Phe Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Gly Leu Pro Ser Ser Ile Glu Lys Thr Ile Ser Lys Ala Lys Gly
                   325                 330                 335
   Gln Pro Arg Glu Pro Gln Val Tyr Thr Leu Pro Pro Ser Gln Glu Glu
               340                 345                 350
   Met Thr Lys Asn Gln Val Ser Leu Thr Cys Leu Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Tyr Ser Arg Leu Thr Val Asp Lys Ser Arg Trp Gln Glu Gly Asn
   405 410 415
   Val Phe Ser Cys Ser Val Met His Glu Ala Leu His Asn His Tyr Thr
   420 425 430
   Gln Lys Ser Leu Ser Leu Ser Leu Gly Lys
   435 440
<210> 5
   <211> 217
   <212> PRT
   <213> chimeric (rat/human)
<400> 5
   Asp Ile Val Leu Thr Gln Ala Pro Val Leu Ala Val Ser Leu Glu Gln
   1 5 10 15
   Arg Ala Thr Ile Ser Cys Lys Ala Ser Gln Asn Val Asp Asn Arg Gly
   20 25 30
   Val Ser Tyr Val His Trp Tyr Gln Gln Lys Pro Arg Gln Gln Pro Lys
   35 40 45
   Leu Leu Ile Tyr Lys Gly Ser Asn Leu Ala Phe Gly Val Pro Ala Arg
   50 55 60
   Phe Ser Gly Ser Gly Ser Arg Thr Asp Phe Thr Leu Thr Ile Asp Pro
   65 70 75 80
   Val Glu Thr Asp Asp Phe Ala Thr Tyr Tyr Cys Gln Gln Ser Lys Gly
   85 90 95
   His Pro Asp Thr Phe Gly Ala Gly Thr Lys Leu Glu Leu Lys Arg Thr
   100 105 110
   Val Ala Ala Pro Ser Val Phe Ile Phe Pro Pro Ser Asp Glu Gln Leu
   115 120 125
   Lys Ser Gly Thr Ala Ser Val Val Cys Leu Leu Asn Asn Phe Tyr Pro
   130 135 140
   Arg Glu Ala Lys Val Gln Trp Lys Val Asp Asn Ala Leu Gln Ser Gly
   145 150 155 160
   Asn Ser Gln Glu Ser Val Thr Glu Gln Asp Ser Lys Asp Ser Thr Tyr
   165 170 175
   Ser Leu Ser Ser Thr Leu Thr Leu Ser Lys Ala Asp Tyr Glu Lys His
   180 185 190
   Lys Val Tyr Ala Cys Glu Val Thr His Gln Gly Leu Ser Ser Pro Val
   195 200 205
   Thr Lys Ser Phe Asn Arg Gly Glu Cys
   210 215
<210> 6
   <211> 115
   <212> PRT
   <213> rat
<400> 6
   Gln Leu Gln Leu Lys Glu Ser Gly Pro Gly Leu Val Gln Pro Ser Gln
   1 5 10 15
   Thr Leu Ser Leu Thr Cys Thr Val Ser Glu Leu Ser Leu Thr Ser Asn
   20 25 30
   Ser Ile Thr Trp Ile Arg Gln Pro Pro Gly Arg Gly Leu Glu Trp Met
   35 40 45
   Gly Met Ile Trp Ser Asn Gly Asp Thr Asp Tyr Asn Ser Ala Phe Thr
   50 55 60
   Ser Arg Leu Ser Ile Ser Arg Asp Thr Ser Lys Ser Gln Val Phe Leu
   65 70 75 80
   Lys Met Asn Ser Leu Gln Thr Glu Asp Ser Ala Met Tyr Phe Cys Ala
   85 90 95
   Arg Tyr Asn Tyr Tyr Phe Asp Tyr Trp Gly Gln Gly Val Met Val Thr
   100 105 110
   Val Ser Ser
   115
<210> 7
   <211> 115
   <212> PRT
   <213> Artificial
<220>
   <223> HURDIVHv1
<400> 7
   Gln Leu Gln Leu Gln Glu Ser Gly Pro Gly Leu Val Lys Pro Ser Gln
   1 5 10 15
   Thr Leu Ser Leu Thr Cys Thr Val Ser Gly Leu Ser Leu Thr Ser Asn
   20 25 30
   Ser Ile Thr Trp Val Arg Gln Ala Pro Gly Gln Gly Leu Glu Trp Met
   35 40 45
   Gly Met Ile Trp Ser Asn Gly Asp Thr Asp Tyr Asn Ser Ala Phe Thr
   50 55 60
   Ser Arg Phe Thr Ile Ser Arg Asp Asn Ser Lys Asn Thr Leu Tyr Leu
   65 70 75 80
   Gln Met Asn Ser Leu Lys Thr Glu Asp Thr Ala Val Tyr Tyr Cys Ala
   85 90 95
   Arg Tyr Asn Tyr Tyr Phe Asp Tyr Trp Gly Gln Gly Thr Leu Val Thr
   100 105 110
   Val Ser Ser
   115
<210> 8
   <211> 115
   <212> PRT
   <213> Artificial
<220>
   <223> HURDIVHv2
<400> 8
   Gln Leu Gln Leu Gln Glu Ser Gly Pro Gly Leu Val Lys Pro Ser Gln
   1 5 10 15
   Thr Leu Ser Leu Thr Cys Thr Val Ser Gly Leu Ser Leu Thr Ser Asn
   20 25 30
   Ser Ile Thr Trp Val Arg Gln Ala Pro Gly Gln Gly Leu Glu Trp Met
   35 40 45
   Gly Met Ile Trp Ser Asn Gly Asp Thr Asp Tyr Ser Thr Ser Leu Lys
   50 55 60
   Ser Arg Leu Thr Ile Ser Lys Asp Thr Ser Lys Ser Gln Val Val Leu
   65 70 75 80
   Thr Met Thr Asn Met Asp Pro Val Asp Thr Ala Thr Tyr Tyr Cys Ala
   85 90 95
   Arg Tyr Asn Tyr Tyr Phe Asp Tyr Trp Gly Gln Gly Thr Leu Val Thr
   100 105 110
   Val Ser Ser
   115
<210> 9
   <211> 115
   <212> PRT
   <213> Artificial
<220>
   <223> HURDIVHv3
<400> 9
   Gln Leu Gln Leu Gln Glu Ser Gly Pro Gly Leu Val Lys Pro Ser Gln
   1 5 10 15
   Thr Leu Ser Leu Thr Cys Thr Val Ser Gly Leu Ser Leu Thr Ser Asn
   20 25 30
   Ser Ile Thr Trp Val Arg Gln Ala Pro Gly Gln Gly Leu Glu Trp Met
   35 40 45
   Gly Met Ile Trp Ser Asn Gly Asp Thr Asp Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Phe Cys Ala
                   85                  90                  95
   Arg Tyr Asn Tyr Tyr Phe Asp Tyr Trp Gly Gln Gly Thr Leu Val Thr
               100                 105                 110
   Val Ser Ser
           115
<210> 10
   <211> 115
   <212> PRT
   <213> Artificial
<220>
   <223> HURDIVHv4
<400> 10
   Gln Leu Gln Leu Gln Glu Ser Gly Pro Gly Leu Val Lys Pro Ser Gln
   1               5                   10                  15
   Thr Leu Ser Leu Thr Cys Thr Val Ser Gly Leu Ser Leu Thr Ser Asn
               20                  25                  30
   Ser Ile Thr Trp Ile Arg Gln Pro Pro Gly Lys Gly Pro Glu Trp Met
           35                  40                  45
   Gly Met Ile Trp Ser Asn Gly Asp Thr Asp Tyr Asn Ser Ala Phe Thr
       50                  55                  60
   Ser Arg Phe Thr Ile Ser Arg Asp Asn Ser Lys Asn Thr Leu Tyr Leu
   65                  70                  75                  80
   Gln Met Asn Ser Leu Lys Thr Glu Asp Thr Ala Val Tyr Tyr Cys Ala
                   85                  90                  95
   Arg Tyr Asn Tyr Tyr Phe Asp Tyr Trp Gly Gln Gly Thr Leu Val Thr
               100                 105                 110
   Val Ser Ser
           115
<210> 11
   <211> 115
   <212> PRT
   <213> Artificial
<220>
   <223> HURDIVHv5
<400> 11
   Gln Leu Gln Leu Gln Glu Ser Gly Pro Gly Leu Val Lys Pro Ser Gln
   1 5 10 15
   Thr Leu Ser Leu Thr Cys Thr Val Ser Gly Leu Ser Leu Thr Ser Asn
   20 25 30
   Ser Ile Thr Trp Ile Arg Gln Pro Pro Gly Lys Gly Pro Glu Trp Met
   35 40 45
   Gly Met Ile Trp Ser Asn Gly Asp Thr Asp Tyr Ser Thr Ser Leu Lys
   50 55 60
   Ser Arg Leu Thr Ile Ser Lys Asp Thr Ser Lys Ser Gln Val Val Leu
   65 70 75 80
   Thr Met Thr Asn Met Asp Pro Val Asp Thr Ala Thr Tyr Tyr Cys Ala
   85 90 95
   Arg Tyr Asn Tyr Tyr Phe Asp Tyr Trp Gly Gln Gly Thr Leu Val Thr
   100 105 110
   Val Ser Ser
   115
<210> 12
   <211> 115
   <212> PRT
   <213> Artificial
<220>
   <223> HURDIVHv6
<400> 12
   Gln Leu Gln Leu Gln Glu Ser Gly Pro Gly Leu Val Lys Pro Ser Gln
   1 5 10 15
   Thr Leu Ser Leu Thr Cys Thr Val Ser Gly Leu Ser Leu Thr Ser Asn
   20 25 30
   Ser Ile Thr Trp Ile Arg Gln Pro Pro Gly Lys Gly Pro Glu Trp Met
   35 40 45
   Gly Met Ile Trp Ser Asn Gly Asp Thr Asp Tyr Asn Pro Ser Leu Lys
   50 55 60
   Ser Arg Val Thr Ile Ser Val Asp Thr Ser Lys Asn Gln Phe Ser Leu
   65 70 75 80
   Lys Leu Ser Ser Val Thr Ala Ala Asp Thr Ala Val Tyr Phe Cys Ala
   85 90 95
   Arg Tyr Asn Tyr Tyr Phe Asp Tyr Trp Gly Gln Gly Thr Leu Val Thr
   100 105 110
   Val Ser Ser
   115
<210> 13
   <211> 110
   <212> PRT
   <213> rat
<400> 13
   Asp Ile Val Leu Thr Gln Ala Pro Val Leu Ala Val Ser Leu Glu Gln
   1 5 10 15
   Arg Ala Thr Ile Ser Cys Lys Ala Ser Gln Asn Val Asp Asn Arg Gly
   20 25 30
   Val Ser Tyr Val His Trp Tyr Gln Gln Lys Pro Arg Gln Gln Pro Lys
   35 40 45
   Leu Leu Ile Tyr Lys Gly Ser Asn Leu Ala Phe Gly Val Pro Ala Arg
   50 55 60
   Phe Ser Gly Ser Gly Ser Arg Thr Asp Phe Thr Leu Thr Ile Asp Pro
   65 70 75 80
   Val Glu Thr Asp Asp Phe Ala Thr Tyr Tyr Cys Gln Gln Ser Lys Gly
   85 90 95
   His Pro Asp Thr Phe Gly Ala Gly Thr Lys Leu Glu Leu Lys
   100 105 110
<210> 14
   <211> 111
   <212> PRT
   <213> Artificial
<220>
   <223> HURDIVKv4
<400> 14
   Asp Ile Val Leu Thr Gln Ser Pro Ala Thr Leu Ser Leu Ser Pro Gly
   1 5 10 15
   Glu Arg Ala Thr Leu Ser Cys Lys Ala Ser Gln Asn Val Asp Asn Arg
   20 25 30
   Gly Val Ser Tyr Val His Trp Tyr Gln Gln Lys Pro Gly Gln Ala Pro
   35 40 45
   Lys Leu Leu Ile Tyr Lys Gly Ser Asn Leu Ala Phe Gly Val Pro Ala
   50 55 60
   Arg Phe Ser Gly Ser Gly Ser Gly Thr Asp Phe Thr Leu Thr Ile Ser
   65 70 75 80
   Ser Leu Gln Pro Glu Asp Phe Ala Thr Tyr Tyr Cys Gln Gln Ser Lys
   85 90 95
   Gly His Pro Asp Thr Phe Gly Ala Gly Thr Lys Leu Glu Leu Lys
   100 105 110
<210> 15
   <211> 111
   <212> PRT
   <213> Artificial
<220>
   <223> HURDIVKv5
<400> 15
   Asp Ile Val Leu Thr Gln Ser Pro Ala Thr Leu Ser Leu Ser Pro Gly
   1               5                   10                  15
   Glu Arg Ala Thr Ile Ser Cys Lys Ala Ser Gln Asn Val Asp Asn Arg
               20                  25                  30
   Gly Val Ser Tyr Val His Trp Tyr Gln Gln Lys Pro Gly Gln Ala Pro
           35                  40                  45
   Lys Leu Leu Ile Tyr Lys Gly Ser Asn Leu Ala Phe Gly Val Pro Ala
       50                  55                  60
   Arg Phe Ser Gly Ser Gly Ser Gly Thr Asp Phe Thr Leu Thr Ile Ser
   65                  70                  75                  80
   Ser Leu Gln Pro Glu Asp Phe Ala Thr Tyr Tyr Cys Gln Gln Ser Lys
                   85                  90                  95
   Gly His Pro Asp Thr Phe Gly Ala Gly Thr Lys Leu Glu Leu Lys
               100                 105                 110
<210> 16
   <211> 111
   <212> PRT
   <213> Artificial
<220>
   <223> HURDIVKv7
<400> 16
   Asp Ile Val Leu Thr Gln Ser Pro Ala Thr Leu Ser Leu Ser Pro Gly
   1               5                   10                  15
   Glu Arg Ala Thr Leu Ser Cys Lys Ala Ser Gln Asn Val Asp Asn Arg
               20                  25                  30
   Gly Val Ser Tyr Val His Trp Tyr Gln Gln Lys Pro Gly Gln Ala Pro
           35                  40                  45
   Lys Leu Leu Ile Tyr Lys Gly Ser Asn Leu Ala Phe Gly Val Pro Ala
   50 55 60
   Arg Phe Ser Gly Ser Gly Ser Gly Thr Asp Phe Thr Leu Thr Ile Ser
   65 70 75 80
   Ser Leu Gln Pro Glu Asp Phe Ala Thr Tyr Phe Cys Gln Gln Ser Lys
   85 90 95
   Gly His Pro Asp Thr Phe Gly Ala Gly Thr Lys Leu Glu Leu Lys
   100 105 110
<210> 17
   <211> 111
   <212> PRT
   <213> Artificial
<220>
   <223> HURDIVKv8
<400> 17
   Asp Ile Val Leu Thr Gln Ser Pro Ala Thr Leu Ser Leu Ser Pro Gly
   1 5 10 15
   Glu Arg Ala Thr Leu Ser Cys Lys Ala Ser Gln Asn Val Asp Asn Arg
   20 25 30
   Gly Val Ser Tyr Val His Trp Tyr Gln Gln Lys Pro Gly Gln Ala Pro
   35 40 45
   Lys Leu Leu Ile Tyr Lys Gly Ser Asn Leu Ala Phe Gly Val Pro Ser
   50 55 60
   Arg Phe Ser Gly Ser Gly Ser Gly Thr Asp Phe Thr Leu Thr Ile Ser
   65 70 75 80
   Ser Leu Gln Pro Glu Asp Phe Ala Thr Tyr Phe Cys Gln Gln Ser Lys
   85 90 95
   Gly His Pro Asp Thr Phe Gly Ala Gly Thr Lys Leu Glu Leu Lys
   100 105 110
<210> 18
   <211> 111
   <212> PRT
   <213> Artificial
<220>
   <223> HURDIVKv9
<400> 18
   Asp Ile Val Leu Thr Gln Ser Pro Ala Thr Leu Ser Leu Ser Pro Gly
   1               5                   10                  15
   Glu Arg Ala Thr Leu Ser Cys Lys Ala Ser Gln Asn Val Asp Asn Arg
               20                  25                  30
   Gly Val Ser Tyr Val His Trp Tyr Gln Gln Lys Pro Gly Gln Ala Pro
           35                  40                  45
   Lys Leu Leu Ile Tyr Lys Gly Ser Asn Leu Ala Phe Gly Val Pro Ser
       50                  55                  60
   Arg Phe Ser Gly Ser Gly Ser Gly Thr Asp Phe Thr Leu Thr Ile Ser
   65                  70                  75                  80
   Ser Leu Gln Pro Glu Asp Phe Ala Thr Tyr Tyr Cys Gln Gln Ser Lys
                   85                  90                  95
   Gly His Pro Asp Thr Phe Gly Ala Gly Thr Lys Leu Glu Leu Lys
               100                 105                 110
<210> 19
   <211> 111
   <212> PRT
   <213> Artificial
<220>
   <223> HURDIVKv10
<400> 19
   Asp Ile Val Leu Thr Gln Ser Pro Ala Thr Leu Ser Leu Ser Pro Gly
   1               5                   10                  15
   Glu Arg Ala Thr Leu Ser Cys Lys Ala Ser Gln Asn Val Asp Asn Arg
               20                  25                  30
   Gly Val Ser Tyr Val His Trp Tyr Gln Gln Lys Pro Gly Gln Ala Pro
           35                  40                  45
   Lys Ser Leu Ile Tyr Lys Gly Ser Asn Leu Ala Phe Gly Val Pro Ser
       50                  55                  60
   Arg Phe Ser Gly Ser Gly Ser Gly Thr Asp Phe Thr Leu Thr Ile Ser
   65                  70                  75                  80
   Ser Leu Gln Pro Glu Asp Phe Ala Thr Tyr Phe Cys Gln Gln Ser Lys
                   85                  90                  95
   Gly His Pro Asp Thr Phe Gly Ala Gly Thr Lys Leu Glu Leu Lys
               100                 105                 110
<210> 20
   <211> 115
   <212> PRT
   <213> Artificial
<220>
   <223> HUR HuVH v1
<400> 20
   Gln Val Gln Leu Gln Glu Ser Gly Pro Gly Leu Val Lys Pro Ser Glu
   1               5                   10                  15
   Thr Leu Ser Leu Thr Cys Thr Val Ser Glu Leu Ser Leu Thr Ser Asn
               20                  25                  30
   Ser Ile Thr Trp Ile Arg Gln Pro Pro Gly Lys Gly Leu Glu Trp Ile
           35                  40                  45
   Gly Met Ile Trp Ser Asn Gly Asp Thr Asp Tyr Asn Ser Ala Phe Thr
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Tyr Asn Tyr Tyr Phe Asp Tyr Trp Gly Gln Gly Thr Leu Val Thr
               100                 105                 110
   Val Ser Ser
   115
<210> 21
   <211> 115
   <212> PRT
   <213> Artificial
<220>
   <223> HUR HuVH v2
<400> 21
   Gln Val Gln Leu Gln Glu Ser Gly Pro Gly Leu Val Lys Pro Ser Glu
   1 5 10 15
   Thr Leu Ser Leu Thr Cys Thr Val Ser Glu Leu Ser Leu Thr Ser Asn
   20 25 30
   Ser Ile Thr Trp Ile Arg Gln Pro Pro Gly Lys Gly Leu Glu Trp Ile
   35 40 45
   Gly Met Ile Trp Ser Asn Gly Asp Thr Asp Tyr Asn Ser Ala Phe Thr
   50 55 60
   Ser Arg Val Thr Ile Ser Arg Asp Thr Ser Lys Asn Gln Phe Ser Leu
   65 70 75 80
   Lys Leu Ser Ser Val Thr Ala Ala Asp Thr Ala Val Tyr Tyr Cys Ala
   85 90 95
   Arg Tyr Asn Tyr Tyr Phe Asp Tyr Trp Gly Gln Gly Thr Leu Val Thr
   100 105 110
   Val Ser Ser
   115
<210> 22
   <211> 115
   <212> PRT
   <213> Artificial
<220>
   <223> HUR HuVH v3
<400> 22
   Gln Leu Gln Leu Gln Glu Ser Gly Pro Gly Leu Val Lys Pro Ser Glu
   1 5 10 15
   Thr Leu Ser Leu Thr Cys Thr Val Ser Glu Leu Ser Leu Thr Ser Asn
   20 25 30
   Ser Ile Thr Trp Ile Arg Gln Pro Pro Gly Lys Gly Leu Glu Trp Met
   35 40 45
   Gly Met Ile Trp Ser Asn Gly Asp Thr Asp Tyr Asn Ser Ala Phe Thr
   50 55 60
   Ser Arg Val Thr Ile Ser Arg Asp Thr Ser Lys Asn Gln Phe Ser Leu
   65 70 75 80
   Lys Leu Ser Ser Val Thr Ala Ala Asp Thr Ala Val Tyr Tyr Cys Ala
   85 90 95
   Arg Tyr Asn Tyr Tyr Phe Asp Tyr Trp Gly Gln Gly Thr Leu Val Thr
   100 105 110
   Val Ser Ser
   115
<210> 23
   <211> 119<212> PRT
   <213> Artificial
<220><223> AAB67785-1
<400> 23
   Gln Val Gln Leu Gln Glu Ser Gly Pro Gly Leu Val Lys Pro Ser Glu
   1 5 10 15
   Thr Leu Ser Leu Thr Cys Thr Val Ser Gly Gly Ser Ile Ser Ser Tyr
   20 25 30
   Tyr Trp Ser Trp Ile Arg Gln Pro Pro Gly Lys Gly Leu Glu Trp Ile
   35 40 45
   Gly Tyr Ile Tyr Tyr Ser Gly Ser Thr Asn Tyr Asn Pro Ser Leu Lys
   50 55 60
   Ser Arg Val Thr Ile Ser Val Asp Thr Ser Lys Asn Gln Phe Ser Leu
   65 70 75 80
   Lys Leu Ser Ser Val Thr Ala Ala Asp Thr Ala Val Tyr Tyr Cys Ala
   85 90 95
   Arg Ala Glu Ala Ala Ala Pro Tyr Tyr Phe Asp Tyr Trp Gly Gln Gly
   100 105 110
   Thr Leu Val Thr Val Ser Ser
   115
<210> 24
   <211> 111
   <212> PRT
   <213> Artificial
<220>
   <223> HUR HuVK v1
<400> 24
   Glu Ile Val Leu Thr Gln Ser Pro Ala Thr Leu Ser Leu Ser Pro Gly
   1 5 10 15
   Glu Arg Ala Thr Leu Ser Cys Lys Ala Ser Gln Asn Val Asp Asn Arg
   20 25 30
   Gly Val Ser Tyr Val His Trp Tyr Gln Gln Lys Pro Gly Gln Ala Pro
   35 40 45
   Arg Leu Leu Ile Tyr Lys Gly Ser Asn Leu Ala Phe Gly Ile Pro Ala
   50 55 60
   Arg Phe Ser Gly Ser Gly Ser Gly Thr Asp Phe Thr Leu Thr Ile Ser
   65 70 75 80
   Ser Leu Glu Pro Glu Asp Phe Ala Val Tyr Tyr Cys Gln Gln Ser Lys
   85 90 95
   Gly His Pro Asp Thr Phe Gly Gln Gly Thr Lys Val Glu Ile Lys
   100 105 110
<210> 25
   <211> 111
   <212> PRT
   <213> Artificial
<220>
   <223> HUR HuVK v2
<400> 25
   Glu Ile Val Leu Thr Gln Ser Pro Ala Thr Leu Ser Leu Ser Pro Gly
   1               5                   10                  15
   Glu Arg Ala Thr Leu Ser Cys Lys Ala Ser Gln Asn Val Asp Asn Arg
               20                  25                  30
   Gly Val Ser Tyr Val His Trp Tyr Gln Gln Lys Pro Gly Gln Ala Pro
           35                  40                  45
   Arg Leu Leu Ile Tyr Lys Gly Ser Asn Leu Ala Phe Gly Val Pro Ala
       50                  55                  60
   Arg Phe Ser Gly Ser Gly Ser Arg Thr Asp Phe Thr Leu Thr Ile Ser
   65                  70                  75                  80
   Ser Leu Glu Pro Glu Asp Phe Ala Thr Tyr Tyr Cys Gln Gln Ser Lys
                   85                  90                  95
   Gly His Pro Asp Thr Phe Gly Gln Gly Thr Lys Val Glu Ile Lys
               100                 105                 110
<210> 26
   <211> 111
   <212> PRT
   <213> Artificial
<220>
   <223> HUR HuVK v3
<400> 26
   Glu Ile Val Leu Thr Gln Ser Pro Ala Thr Leu Ser Leu Ser Pro Gly
   1               5                   10                  15
   Glu Arg Ala Thr Ile Ser Cys Lys Ala Ser Gln Asn Val Asp Asn Arg
               20                  25                  30
   Gly Val Ser Tyr Val His Trp Tyr Gln Gln Lys Pro Gly Gln Gln Pro
           35                  40                  45
   Lys Leu Leu Ile Tyr Lys Gly Ser Asn Leu Ala Phe Gly Val Pro Ala
       50                  55                  60
   Arg Phe Ser Gly Ser Gly Ser Arg Thr Asp Phe Thr Leu Thr Ile Ser
   65                  70                  75                  80
   Ser Leu Glu Pro Glu Asp Phe Ala Thr Tyr Tyr Cys Gln Gln Ser Lys
                   85                  90                  95
   Gly His Pro Asp Thr Phe Gly Gln Gly Thr Lys Val Glu Ile Lys
               100                 105                 110
<210> 27
   <211> 107
   <212> PRT
   <213> Artificial
<220>
   <223> CAD43025
<400> 27
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Tyr
               20                  25                  30
   Val Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Ser Asn Asn Trp Pro Gln
                   85                  90                  95
   Thr Phe Gly Gln Gly Thr Lys Val Glu Ile Lys
   100 105
<210> 28
   <211> 313
   <212> PRT
   <213> Homo sapiens
<400> 28
   Asp Lys Cys Lys Glu Arg Glu Glu Lys Ile Ile Leu Val Ser Ser Ala
   1 5 10 15
   Asn Glu Ile Asp Val Arg Pro Cys Pro Leu Asn Pro Asn Glu His Lys
   20 25 30
   Gly Thr Ile Thr Trp Tyr Lys Asp Asp Ser Lys Thr Pro Val Ser Thr
   35 40 45
   Glu Gln Ala Ser Arg Ile His Gln His Lys Glu Lys Leu Trp Phe Val
   50 55 60
   Pro Ala Lys Val Glu Asp Ser Gly His Tyr Tyr Cys Val Val Arg Asn
   65 70 75 80
   Ser Ser Tyr Cys Leu Arg Ile Lys Ile Ser Ala Lys Phe Val Glu Asn
   85 90 95
   Glu Pro Asn Leu Cys Tyr Asn Ala Gln Ala Ile Phe Lys Gln Lys Leu
   100 105 110
   Pro Val Ala Gly Asp Gly Gly Leu Val Cys Pro Tyr Met Glu Phe Phe
   115 120 125
   Lys Asn Glu Asn Asn Glu Leu Pro Lys Leu Gln Trp Tyr Lys Asp Cys
   130 135 140
   Lys Pro Leu Leu Leu Asp Asn Ile His Phe Ser Gly Val Lys Asp Arg
   145 150 155 160
   Leu Ile Val Met Asn Val Ala Glu Lys His Arg Gly Asn Tyr Thr Cys
   165 170 175
   His Ala Ser Tyr Thr Tyr Leu Gly Lys Gln Tyr Pro Ile Thr Arg Val
   180 185 190
   Ile Glu Phe Ile Thr Leu Glu Glu Asn Lys Pro Thr Arg Pro Val Ile
   195 200 205
   Val Ser Pro Ala Asn Glu Thr Met Glu Val Asp Leu Gly Ser Gln Ile
   210 215 220
   Gln Leu Ile Cys Asn Val Thr Gly Gln Leu Ser Asp Ile Ala Tyr Trp
   225 230 235 240
   Lys Trp Asn Gly Ser Val Ile Asp Glu Asp Asp Pro Val Leu Gly Glu
   245 250 255
   Asp Tyr Tyr Ser Val Glu Asn Pro Ala Asn Lys Arg Arg Ser Thr Leu
   260 265 270
   Ile Thr Val Leu Asn Ile Ser Glu Ile Glu Ser Arg Phe Tyr Lys His
   275 280 285
   Pro Phe Thr Cys Phe Ala Lys Asn Thr His Gly Ile Asp Ala Ala Tyr
   290 295 300
   Ile Gln Leu Ile Tyr Pro Val Thr Asn
   305 310
<210> 29
   <211> 107
   <212> PRT
   <213> Homo sapiens
<400> 29
<210> 30
   <211> 327
   <212> PRT
   <213> Homo sapiens
<400> 30
   Ala Ser Thr Lys Gly Pro Ser Val Phe Pro Leu Ala Pro Cys Ser Arg
   1 5 10 15
   Ser Thr Ser Glu Ser Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Lys Thr
   65 70 75 80
   Tyr Thr Cys Asn Val Asp His Lys Pro Ser Asn Thr Lys Val Asp Lys
   85 90 95
   Arg Val Glu Ser Lys Tyr Gly Pro Pro Cys Pro Ser Cys Pro Ala Pro
   100 105 110
   Glu Phe Leu Gly Gly Pro Ser Val Phe Leu Phe Pro Pro Lys Pro Lys
   115 120 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys
                   325
<210> 31
   <211> 330
   <212> PRT
   <213> Homo sapiens
<400> 31
<210> 32
   <211> 442
   <212> PRT
   <213> Artificial
<220>
   <223> heavy chain of DEI 5/8
<400> 32
   Gln Leu Gln Leu Gln Glu Ser Gly Pro Gly Leu Val Lys Pro Ser Gln
   1               5                   10                  15
   Thr Leu Ser Leu Thr Cys Thr Val Ser Gly Leu Ser Leu Thr Ser Asn
               20                  25                  30
   Ser Ile Thr Trp Ile Arg Gln Pro Pro Gly Lys Gly Pro Glu Trp Met
           35                  40                  45
   Gly Met Ile Trp Ser Asn Gly Asp Thr Asp Tyr Ser Thr Ser Leu Lys
       50                  55                  60
   Ser Arg Leu Thr Ile Ser Lys Asp Thr Ser Lys Ser Gln Val Val Leu
   65                  70                  75                  80
   Thr Met Thr Asn Met Asp Pro Val Asp Thr Ala Thr Tyr Tyr Cys Ala
                   85                  90                  95
   Arg Tyr Asn Tyr Tyr Phe Asp Tyr Trp Gly Gln Gly Thr Leu Val Thr
               100                 105                 110
   Val Ser Ser Ala Ser Thr Lys Gly Pro Ser Val Phe Pro Leu Ala Pro
           115                 120                 125
   Cys Ser Arg Ser Thr Ser Glu Ser Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Lys Thr Tyr Thr Cys Asn Val Asp His Lys Pro Ser Asn Thr Lys
           195                 200                 205
   Val Asp Lys Arg Val Glu Ser Lys Tyr Gly Pro Pro Cys Pro Ser Cys
       210                 215                 220
   Pro Ala Pro Glu Phe Leu Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser Gln Glu Asp Pro Glu Val Gln Phe Asn Trp
               260                 265                 270
   Tyr Val Asp Gly Val Glu Val His Asn Ala Lys Thr Lys Pro Arg Glu
   275 280 285
   Glu Gln Phe Asn Ser Thr Tyr Arg Val Val Ser Val Leu Thr Val Leu
   290 295 300
   His Gln Asp Trp Leu Asn Gly Lys Glu Tyr Lys Cys Lys Val Ser Asn
   305 310 315 320
   Lys Gly Leu Pro Ser Ser Ile Glu Lys Thr Ile Ser Lys Ala Lys Gly
   325 330 335
   Gln Pro Arg Glu Pro Gln Val Tyr Thr Leu Pro Pro Ser Gln Glu Glu
   340 345 350
   Met Thr Lys Asn Gln Val Ser Leu Thr Cys Leu Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Tyr Ser Arg Leu Thr Val Asp Lys Ser Arg Trp Gln Glu Gly Asn
   405 410 415
   Val Phe Ser Cys Ser Val Met His Glu Ala Leu His Asn His Tyr Thr
   420 425 430
   Gln Lys Ser Leu Ser Leu Ser Leu Gly Lys
   435 440
<210> 33
   <211> 218
   <212> PRT
   <213> Artificial
<220>
   <223> light chain of DEI 5/8
<400> 33
   Asp Ile Val Leu Thr Gln Ser Pro Ala Thr Leu Ser Leu Ser Pro Gly
   1               5                   10                  15
   Glu Arg Ala Thr Leu Ser Cys Lys Ala Ser Gln Asn Val Asp Asn Arg
               20                  25                  30
   Gly Val Ser Tyr Val His Trp Tyr Gln Gln Lys Pro Gly Gln Ala Pro
           35                  40                  45
   Lys Leu Leu Ile Tyr Lys Gly Ser Asn Leu Ala Phe Gly Val Pro Ser
       50                  55                  60
   Arg Phe Ser Gly Ser Gly Ser Gly Thr Asp Phe Thr Leu Thr Ile Ser
   65                  70                  75                  80
   Ser Leu Gln Pro Glu Asp Phe Ala Thr Tyr Phe Cys Gln Gln Ser Lys
                   85                  90                  95
   Gly His Pro Asp Thr Phe Gly Ala Gly Thr Lys Leu Glu Leu Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215